(19) Europäisches Patentamt
European Patent Office
Office européen des brevets

(11) Veröffentlichungsnummer : **0 346 281 B1**

# (12) EUROPÄISCHE PATENTSCHRIFT

(45) Veröffentlichungstag der Patentschrift : **17.06.92 Patentblatt 92/25**

(51) Int. Cl.[5] : **C07C 271/60,** C07C 259/06, A61K 31/27

(21) Anmeldenummer : **89810405.4**

(22) Anmeldetag : **30.05.89**

## (54) Carbamoylierte Hydroxamsäurederivate und Verfahren zu ihrer Herstellung.

(30) Priorität : **08.06.88 CH 2181/88**

(43) Veröffentlichungstag der Anmeldung : **13.12.89 Patentblatt 89/50**

(45) Bekanntmachung des Hinweises auf die Patenterteilung : **17.06.92 Patentblatt 92/25**

(84) Benannte Vertragsstaaten : **AT BE CH DE ES FR GB GR IT LI LU NL SE**

(56) Entgegenhaltungen :
**EP-A- 0 271 443**
**EP-A- 0 300 969**
**WO-A-85/03290**
**WO-A-86/03747**

(73) Patentinhaber : **CIBA-GEIGY AG**
**Klybeckstrasse 141**
**CH-4002 Basel (CH)**

(72) Erfinder : **Peter, Heinrich, Dr.**
**Bündtenweg 69**
**CH-4102 Binningen (CH)**
Erfinder : **Moerker, Théophile**
**Ergolzstrasse 72**
**CH-4414 Füllinsdorf (CH)**

EP 0 346 281 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Carbamoylderivate von Hydroxamsäuren, insbesondere von Trihydroxamsäuren, welche unter der Bezeichnung Ferrioxamine und speziell Desferrioxamine als Stoffwechselprodukte von Mikroorganismen, insbesondere Actinomyceten, bekannt sind. Darunter betrifft die Erfindung vor allem die von Desferrioxamin B abgeleiteten O-Carbamoylderivate der allgemeinen Formel I,

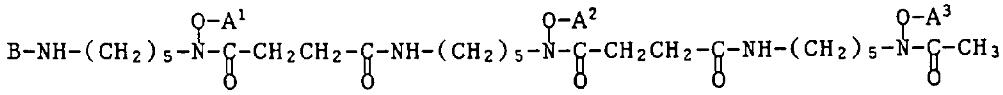

(I)

in welcher mindestens einer der Reste $A^1$, $A^2$ und $A^3$ für einen Carbamoylrest der Teilformel -CO-NH-Alk-CO-O-$R_a^1$ (II) steht, worin $R_a^1$ $C_1$-$C_4$-Alkyl oder $C_2$-$C_4$-Alkenyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, und die übrigen der Symbole $A^1$, $A^2$ und $A^3$ unabhängig voneinander für Wasserstoff oder einen von einer Carbonsäure abgeleiteten Acylrest Ac stehen, und B für Wasserstoff oder einen Acylrest Ac steht, welcher von Alkyl-O-($CH_2$-$CH_2$-O-)$_q$CO-, worin q eine Zahl grösser als 5 bedeutet, verschieden ist, sowie Salze von solchen Verbindungen mit salzbildenden Eigenschaften.

Die Erfindung betrifft auch Verfahren zur Herstellung der oben genannten Verbindungen, sowie diese Verbindungen enthaltende pharmazeutische Zusammensetzungen und Verfahren zu ihrer Herstellung; ferner auch die therapeutische Anwendung dieser Verbindungen und diese enthaltender pharmazeutischer Zusammensetzungen bei Warmblütern einschliesslich des Menschen.

Die EP-A-271443 gehört zum Stand der Technik gemäss Art. 54(3) EPUe und beschreibt Verbindungen der obigen Formel I, worin B einen Carbamoylrest der obigen Formel II bedeutet. Im Unterschied dazu können in der vorliegenden Anmeldung zwar die Reste $A^1$, $A^2$ und $A^3$, nicht aber der Rest B einen solchen Carbamoylrest der Formel II bedeuten.

Die EP-A-300969 beschreibt Derivate von Desferrioxamin B, worin der dem Rest B gemäss der obigen Formel I entsprechende Rest ein langkettiger Rest der Formel Alkyl-O-($CH_2$-$CH_2$-O)$_n$-C(=O)-(NH-$SO_2$)$_m$- ist, worin n für mindestens 9 und m für 0 oder 1 stehen.

Die WO-A-86/03747 beschreibt Verbindungen, worin in der obigen Formel I mindestens einer der Reste B, $A^1$, $A^2$ und $A^3$ den Acylrest eines Kohlensäuremonoesters darstellt.

Die WO-A-85/03290 beschreibt Derivate von Desferrioxamin B, die jedoch keinen Carbamoylrest der obigen Formel II enthalten.

Desferrioxamin B, der Grundstoff der Acylate der vorliegenden Erfindung, ist bereits längere Zeit bekannt (H. Bickel, H. Keberle und E. Vischer: Helv.Chim.Acta <u>46</u>, 1385-9 [1963]). Seine chemische Struktur entspricht der Formel

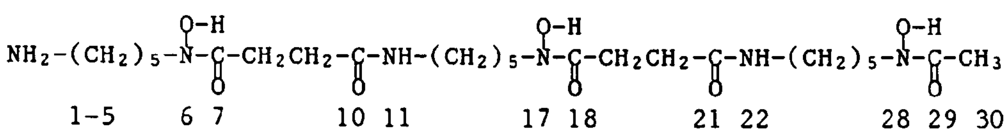

(A)

und wird im Einklang mit der Regel C-O6 (Austausch-Nomenklatur der offiziellen IUPAC-Nomenklatur) mit den systematischen Namen 6,17,28-Trihydroxy-7,10,18,21,29-pentaoxo-6,11,17,22,28-pentaazatriacontylamin bezeichnet. Der Einfachheit halber werden jedoch nachfolgend die Namen der Acylate vom trivialen Namen abgeleitet, wobei die Lage einzelner Acylreste jeweils auf den Aminostickstoff N bzw. die als O, O′ und O″ bezeichneten Sauerstoffatome der Hydroxylgruppen in Stellungen 6, 17, bzw. 28 bezogen wird.

Zu den markantesten Eigenschaften von Desferrioxamin B und seinen Additionssalzen, die mit einem Aequivalent Säure gebildet werden, gehört die Fähigkeit, sich zu stabilen Chelat-artigen Metallkomplexen, insbesondere mit trivalenten Metallionen, wie Chrom(III)-, Aluminium- und in erster Linie Eisen(III)-Ionen, zu verbinden. Dies verleiht Desferrioxamin B die wertvolle pharmakologische Wirksamkeit, die Ablagerung eisenhaltiger Pigmente im Gewebe zu verhindern und bei bestehenden Eisenablagerungen im Organismus eine Ausscheidung des Eisens zu bewirken, z.B. bei Hämochromatose, Hämosiderose, Lebercirrhose und Vergiftungen mit Verbindungen des dreiwertigen Eisens. Die breite therapeutische Anwendung von Desferrioxamin B und seinen Salzen (z.B. insbesondere vom Methansulfonat) erstreckt sich deshalb allgemein auf Krankheiten und Krankhafte Zustände des menschlichen Körpers (sowie des Körpers anderer Warmblüter), welche mit übermässiger Belastung des Organismus mit Eisen(III)-Ionen ($Fe^{+++}$-Ionen) einhergehen, wie Thalassämie major, Sichelzell-Anämie, sideroachrestische Anämie, aplastische Anämie und weitere anämische Formen, in welchen Hämosiderose (d.h. eine lokale oder allgemeine Erhöhung der Eisenvorräte in sonst unbeschädigten Körpergeweben) eine Rolle spielt. Zu diesem Typus gehören auch krankhafte Zustände, die sich in Patienten nach mehrmaligen Bluttransfusionen oder mehrfach wiederholter Dialyse-Behandlung bei fehlender oder geschädigter Nierenfunktion entwickeln. Dank den komplexbildenden Eigenschaften weist Desferrioxamin B eine bedeutende Wirksamkeit bei Erkrankungen durch Eisen(III)-abhängige Mikroorganismen und Parasiten auf, wie insbesondere bei Malaria, die nicht nur in der Humanmedizin, sondern auch in der Tiermedizin von grosser Bedeutung ist. Sie können daher, vorzugsweise in Kombination mit anderen Malariamitteln, z.B. Chloroquin, zur Therapie von Malaria, sogar bereits in ihrer latenten Phase, d.h. gegen die Leberformen der Malariaerreger, eingesetzt werden. Auch die Komplex-Bildung mit anderen dreiwertigen Metallen kann zu deren Ausscheidung aus dem Organismus genützt werden, z.B. zur Entfernung von Aluminium bei der Dialyse-Encephalopathie und Osteomalacia, sowie bei der Alzheimer Krankheit.

Als ein schwerwiegender Nachteil erweist sich jedoch die für manche Anwendungsformen, z.B. die Behandlung von Malaria, zu kurze Wirkungsdauer von Desferrioxamin B sowie die Tatsache, dass Desferrioxamin B und seine Salze bei oraler Gabe nur eine geringe, unzureichende Wirksamkeit aufweisen und bei allen obengenannten Anwendungsmöglichkeiten eine parenterale Verabreichungsform benötigen. So ist z.B. als eine besonders wirksame Methode empfohlen, die Wirksubstanz mittels einer langsamen (8- bis 12stündigen) subkutanen Infusion zu verabreichen, was aber entweder eine Hospitalisierung des Patienten oder, bei ambulanter Behandlung, die Anwendung einer tragbaren mechanischen Vorrichtung, wie einer durch elektrischen Antrieb betätigten Infusionsspritze, bedingt. Abgesehen von ihrer Umständlichkeit sind solche Lösungen mit hohen Behandlungskosten behaftet, was ihre Anwendung stark einschränkt. Insbesondere wird eine umfassende Behandlung der Thalassämie in den Ländern des Mittelmeerraums, des Mittleren Ostens, Indiens und Südostasiens, der Malaria weltweit und der Sichelzell-Anämie in den afrikanischen Ländern verunmöglicht. Diese weit verbreiteten Krankheiten stellen weiterhin ein schwerwiegendes Problem für das Gesundheitswesen in diesen Ländern dar und machen die Suche nach einer einfacheren und billigeren Therapie, vorzugsweise mittels eines oral wirksame Präparats und/oder mittels eines Präparats mit verlängerter Wirkungsdauer, zur vordringlichen Aufgabe auf diesem Gebiet.

Aufgrund theoretischer Vorstellungen ist anzunehmen, dass zur Chelatisierung von Metallionen und somit zur therapeutisch anwendbaren Metallkomplex-Bildung die freien Hydroxylgruppen des Desferrioxamins B den wesentlichsten strukturellen Beitrag leisten. Wenn man sie aber durch Carbamoylierung blockiert, ist zu erwarten, dass solche Carbamoylderivate, wenn überhaupt, dann nur in einem sehr geringen Masse komplexbildende Eigenschaften und demzufolge auch die wesentliche Voraussetzung für die therapeutische Anwendung besitzen können.

Im Gegensatz zu diesen Ueberlegungen wurde nun gefunden, das in denselben Indikationen, in welchen bisher Desferrioxamin B, z.B. in Form des eingeführten Handelspräparats Desferal®, nur als parenterale Darreichungsform wirksam war, die oben charakterisierte neuartige Klasse von Carbamoylderivaten der Formel I analoge Wirkungen bei oraler Verabreichung aufweist.

Die Verbindungen der Formel I bewirken überraschenderweise sowohl bei parenteraler als auch bei oraler Verabreichung an Warmblüter einschliesslich des Menschen in einer Dosierung zwischen etwa 4 und 100, insbesondere zwischen etwa 5 und 40 μmol/kg eine signifikant erhöhte Ausscheidung von Metallen, wie insbesondere Eisen. Dies kann z.B. im Tiermodell, wie beispielsweise an eisenüberladenen Affen, bei denen die Eisenausscheidung im Urin und in den Faeces bestimmt wird, oder an der nicht-eisenüberladenen Gallenfistelratte experimentell gezeigt werden. Dabei ist insbesondere die langsam einsetzende und lang andauernde Wirkung (Depot-effekt) dieser Verbindungen bemerkenswert. Die tägliche Dosis bei Verabreichung an einen Warmblüter von etwa 70 kg Körpergewicht beträgt etwa 0,5 g bis etwa 5 g, z.B. 2 g Wirkstoff.

In den erfindungsgemässen Verbindungen der Formel I können sich die Symbole $A^1$, $A^2$ und $A^3$ voneinander auch innerhalb ein und derselben Kategorie unterscheiden: So kann jedes dieser Symbole einen anderen Carbamoylrest (II) bedeuten. Vorzugsweise haben aber alle 3 Symbole jeweils dieselbe Bedeutung und stellen einen Carbamoylrest der Formel (II) dar.

Der N-substituierte Carbamoylrest (II) ist wie folgt näher charakterisiert:

$C_1$-$C_4$-Alkyl $R_a^1$ ist vorzugsweise linear, wie insbesondere Methyl und vor allem Ethyl. $C_2$-$C_4$-Alkylen $R_a^1$ ist in erster Linie Allyl.

$C_1$-$C_7$-Alkylen Alk ist vorzugsweise $C_1$-$C_5$-Alkylen und kann beliebig verzweigt sein, wobei seine 2 freien Valenzen von zwei verschiedenen C-Atomen oder ein und demselben C-Atom ausgehen können. Substituiertes Alkylen kann einen der eingangs genannten Substituenten an einem beliebigen C-Atom tragen. Bevorzugt sind lineare Alkylenreste, die die freien Valenzen an beiden endständigen C-Atomen haben, wie Tri- bis Heptamethylen und insbesondere Ethylen. Sie können auch, vorzugsweise an ihren endständigen C-Atomen, einen Substituenten tragen, wie insbesondere Carbamoyl oder $C_1$-$C_4$-Alkoxycarbonyl (vor allem Methoxy- und Ethoxycarbonyl) oder eine primäre Aminogruppe; die zwei erstgenannten Substituenten knüpfen sich vorzugsweise an das N-terminale Ende des Alkylenrestes (d.h. an jenes, das mit der benachbarten Aminogruppe verbunden ist) an, der letztgenannte befindet sich vorzugsweise am C-terminalen Ende, d.h. an demjenigen, das mit der nachfolgenden Carbonylgruppe verbunden ist. Bevorzugt sind auch linear oder höchstens einmal verzweigte Alkylenreste, deren beide freie Valenzen von demselben C-Atom, und zwar vorzugsweise einem endständigen, ausgehen, d.h. lineare oder einmal verzweigte $C_1$-$C_5$-1,1-Alkylidenreste, wie in erster Linie Methylen, aber auch Ethyliden, 1,1-Propyliden usw. In Uebereinstimmung mit der obigen Definition des Begriffes "Alkylen" können die 1,1-Alkylidenreste auch als 1,1-Alkylenreste bezeichnet werden. Diese können auch einen der eingangs genannten Substituenten tragen, vorzugsweise am endständigen C-Atom , wie z.B. eine freie Aminogruppe (insbesondere im 4-Amino-1,1-butyliden oder 5-Amino-1,1-pentyliden), Carbamoyl oder $C_1$-$C_4$-Alkoxycarbonyl, wie einen der obengenannten $C_1$-$C_4$-Alkoxycarbonylreste [ insbesondere im 2-Carbamoyl-1,1-ethyliden, 2-(Methoxy- oder Ethoxy)-carbonyl-1,1-ethyliden oder entsprechenden 3-substituierten 1,1-Propyliden-Resten], ferner auch eine Hydroxyl- oder $C_1$-$C_4$-Alkanoyloxy (insbesondere Acetoxy)-Gruppe, welche sich vorzugsweise in 2-Stellung befindet (insbesondere im 2-Hydroxy-1,1-ethyliden und 2-Hydroxy-1,1-propyliden und entsprechenden acylierten, vor allem acetylierten Resten). Die cyclischen Substituenten befinden sich vorzugsweise am Methylen oder auch in 2-Stellung des Ethylidenrestes.

Ein besonders bevorzugter Alkylenrest ist ein solcher, der zusammen mit der benachbarten Amino- und Carbonylgruppe durch die Teilformel -NH-Alk-CO- bzw. durch das Symbol -AAA- bezeichnet wird und welcher einerseits der allgemeinen Definition des Rests Alk, andererseits der Struktur gewisser in der Natur geläufiger $\alpha$-Aminosäuren (in Form ihrer individuellen optischen Isomeren oder deren Gemische, insbesondere des razemischen Gemisches) entspricht. Der allgemeinen Konvention nach ist eine solche "geläufige $\alpha$-Aminosäure" ("common $\alpha$-amino acid") eine der 20 Aminosäuren, welche in der Natur regelmässig als elementare Bausteine von Peptiden und Proteinen auftreten; sie werden üblicherweise im Einklang mit der internationalen Konvention durch eine Dreibuchstaben-Kurzform bezeichnet. Von dieser Gruppe sind im vorliegenden Fall infolge der Begrenzung durch die Allgemeine Definition des Restes Alk z.B. die folgenden Säuren aus der erfindungsgemässen Bedeutung des Symbols AAA ausgenommen: Arginin (Arg), Cystein (Cys), Histidin (His) und Methionin (Met). Der obengenannte Rest -NH-Alk-CO- kann somit z.B. der bivalente Rest einer der folgenden Aminosäuren sein: Glycin (Gly), Alanin (Ala), Valin (Val), Leucin (Leu), Isoleucin (Ile), Phenylalanin (Phe), Serin (Ser), Threonin (Thr), Tryptophan (Trp), Tyrosin (Tyr), Asparagin (Asn), Glutamin (Gln) und Lysin (Lys). Entsprechende besonders bevorzugte Reste der Teilformel II sind demnach durch die Teilformel -CO-AAA-O-$R_a^1$ (IIA) dargestellt, worin $R_a^1$ die obengenannten allgemeinen und bevorzugten Bedeutungen hat und -AAA- einen durch die obige allgemeine Definition von Alk begrenzten Rest bestimmter geläufiger $\alpha$-Aminosäuren in Form eines individuellen optischen Isomeren oder eines Gemisches davon bedeutet. Als optisch individuelle Form ist das "natürliche" Isomere der L-Reihe bevorzugt, als Isomerengemische sind solche bevorzugt, in welchen beiden Antipoden in gleicher Menge vertreten sind, d.h. Razemate.

In erster Linie steht -AAA- für den Glycinrest (-Gly-) und der ganze Rest der Formel II ist als -CO-Gly-O-$R_a^1$ (IIB) hervorzuheben, in welchem $R_a^1$ vor allem Methyl oder insbesondere Ethyl bedeutet.

Bevorzugterweise steht Alk für Ethylen, d.h. -AAA- ist der bivalente Rest von $\beta$-Alanin.

Der Acylrest Ac leitet sich von Hydrocarbylcarbonsäuren oder Monoestern der Kohlensäure ab und entspricht der Formel Z-C(=O)-, worin Z entweder für Wasserstoff steht (und somit den Formylrest bildet) oder Hydrocarbyl R° darstellt (und somit den Rest einer gegebenenfalls substituierten acyclischen, carbocyclischen, carbocyclisch-acyclischen, heterocyclischen oder heterocyclisch-acyclischen Monocarbonsäure bildet) oder Diniederalkylamino bedeutet (und somit für den Acylrest von N-Diniederalkyl-carbaminsäure steht) oder aber bevorzugterweise Hydrocarbyloxy R°-O- ist (und den Acylrest einer einfach veresterten Kohlensäure darstellt).

Der Hydrocarbylrest (Kohlenwasserstoffrest) R° ist ein acyclischer (aliphatischer), carbocyclischer oder carbocyclisch-acyclischer Kohlenwasserstoffrest, der insgesamt vorzugsweise höchstens 40, insbesondere höchstens 20 und hauptsächlich höchstens 9, Kohlenstoffatome hat und gesättigt oder ungesättigt, unsubstituiert oder substituiert sein kann. Er kann auch anstelle von einem, zwei oder mehreren Kohlenstoffatomen gleiche oder verschiedene Heteroatome, wie insbesondere Sauerstoff, Schwefel und Stickstoff, im acyclischen und/oder cyclischen Teil enthalten; im letzteren Fall wird er als ein heterocyclischer Rest (Heterocyclylrest) oder ein heterocyclisch-acyclischer Rest bezeichnet.

Ungesättigte Reste sind solche, die eine oder mehrere Mehrfachbindungen (Doppel- und/oder Dreifachbindungen) enthalten. Cyclische Reste, worin mindestens ein 6gliedriger carbocyclischer oder ein 5- bis 8gliedriger heterocyclischer Ring die maximale Anzahl nichtkumulierter Doppelbindungen enthält, werden als aromatisch bezeichnet. Carbocyclische Reste, worin mindestens ein Ring als ein 6gliedriger aromatischer Ring (d.h. Benzolring) vorliegt, werden als Arylreste bezeichnet.

Wenn nicht anders angegeben, enthalten in der vorliegenden Offenbarung mit dem Präfix "Nieder" bezeichnete organische Reste höchstens 7, vorzugsweise höchstens 4 Kohlenstoffatome.

Ein acyclischer Kohlenwasserstoffrest ist insbesondere ein Alkyl-, Alkenyl-, Alkadienyl- oder Alkynylrest, welcher verzweigt oder vorzugsweise linear ist.

Ein carbocyclischer Kohlenwasserstoffrest ist insbesondere ein mono-, bi-oder polycyclischer Cycloalkyl-, Cycloalkenyl- oder Cycloalkadienylrest, oder ein entsprechender, aromatische Ringe enthaltender Arylrest, vorzugsweise ein solcher mit höchstens 12 Ringkohlenstoffatomen und 5-bis 7, vor allem 6gliedrigen Ringen. Carbocyclisch-acyclische Reste sind solche, in welchen ein acyclischer Rest, insbesondere einer mit höchstens 7, vorzugsweise höchstens 4 Kohlenstoffatomen, wie vor allem Methyl, Ethyl und Vinyl, einen oder mehrere carbocyclische, gegebenenfalls aromatische Reste der obigen Definition trägt.

Ein Arylrest ist in erster Linie ein Phenylrest, ferner ein Naphthylrest, wie 1- oder 2-Naphthyl, ein Biphenylylrest, wie insbesondere 4-Biphenylyl, weiter auch ein Anthryl-, Fluorenyl-oder Azulenylrest, sowie ihre Analogen mit einem oder mehreren gesättigten Ringen. Bevorzugte Aryl-niederalkyl- und niederalkenyl-Reste sind z.B. Phenylniederalkyl oder Phenyl-niederalkenyl mit endständigem Phenylrest, wie z.B. Benzyl, Phenethyl, bzw. Styryl und Cinnamyl, ferner auch o-, m- und p-Tolyl.

Heterocyclische Reste, einschliesslich heterocyclisch-acyclische Reste, sind insbesondere monocyclische, aber auch bi- oder polycyclische, aza-, thia-, oxa-, thiaza-, oxaza-, diaza-, triaza- oder tetraza-Reste aromatischen Charakters, sowie entsprechende partiell oder in erster Linie ganz gesättigte heterocyclische Reste dieser Art, wobei solche Reste gegebenenfalls, z.B. wie die obgenannten carbocyclischen oder Arylreste, weitere acyclische, carbocyclische oder heterocyclische Reste tragen können und/oder durch funktionelle Gruppen mono-, di- oder polysubstituiert sein können. Der acyclische Teil in heterocyclisch-acyclischen Resten hat z.B. die für die entsprechenden carbocyclisch-acyclischen Reste gegebene Bedeutung. Sofern sich ein Heterocyclyl als ein direkter Substituent R° im Symbol Z am Sauerstoff befindet, so muss seine freie Valenz von einem seiner C-Atome ausgehen.

Wie bereits erwähnt wurde, kann ein Hydrocarbyl (einschliesslich eines Heterocyclyls) R° durch einen, zwei oder mehrere gleichartige oder verschiedenartige Substituenten (funktionelle Gruppen) substituiert sein; die folgenden Substituenten kommen insbesondere in Betracht: freie, veretherte und veresterte Hydroxylgruppen; Mercapto- sowie Niederalkylthio- und gegebenenfalls substituierte Phenylthiogruppen; Halogenatome, wie Chlor und Fluor, aber auch Brom und Iod; Oxogruppen, welche in der Form von Formyl- (d.h. Aldehydo-) und Keto-gruppen, auch als entsprechende Acetale bzw. Ketale vorliegen; Azido- und Nitrogruppen; primäre, sekundäre und vorzugsweise tertiäre Aminogruppen, durch konventionelle Schutzgruppen geschützte primäre oder sekundäre Aminogruppen, Acylaminogruppen und Diacylaminogruppen, sowie gegebenenfalls funktionell abgewandelte Sulfogruppen, wie Sulfamoyl- oder in Salzform vorliegende Sulfogruppen. Alle diese funktionellen Gruppen dürfen sich nicht am C-Atom befinden, von dem die freie Valenz zum Sauerstoff ausgeht, vorzugsweise sind sie von dieser freien Valenz (und somit vom Heteroatom) durch 2 oder auch mehrere C-Atome getrennt. Der Hydrocarbylrest kann auch freie und funktionell abgewandelte Carboxylgruppen, wie in Salzform vorliegende oder veresterte Carboxylgruppen, gegebenenfalls einen oder zwei Kohlenwasserstoffreste tragende Carbamoyl-, Ureidocarbonyl- oder Guanidinocarbonylgruppen, und Cyangruppen tragen.

Eine als Substituent im Hydrocarbyl vorliegende veretherte Hydroxylgruppe ist z.B. eine Niederalkoxygruppe, wie die Methoxy-, Ethoxy- oder tert-Butoxygruppe, welche auch, durch Halogenatome, insbesondere in 2-Stellung, oder durch Niederalkoxyreste, insbesondere in 2-Stellung, wie im 2-Methoxyethoxyrest, substituiert sein kann. Eine besonders bevorzugte Ausgestaltung der veretherten Hydroxylgruppen liegt in Oxaalkylresten vor, in welchen ein, vorzugsweise lineares, Alkyl anstelle mehrerer C-Atome Sauerstoffatome enthält, die vorzugsweise durch mehrere (vor allem 2) C-Atome voneinander getrennt sind. Bevorzugte Oxaalkylreste enthalten eine Gruppe der Formel $(O\text{-}CH_2CH_2\text{-})_n$, worin n = 1 bis 4, insbesondere 1, 2 oder 3, ist, und enthalten bis zu 19, insbesondere bis zu 16 und vorzugsweise bis zu 13 Kettenglieder (Summe aller Kohlenstoff-und Sauerstoffatome).

Eine als Substituent im Hydrocarbyl vorliegende veresterte Hydroxylgruppe trägt einen Acylrest Ac° mit höchstens 12 C-Atomen, welcher auch innerhalb dieser Gesamtzahl der C-Atome analog wie der Rest Ac substituiert sein kann, oder ist durch eine im Hydrocarbyl auch anwesende Carboxylgruppe lactonisiert.

Eine als Substituent im Hydrocarbyl vorliegende veresterte Carboxylgruppe ist eine solche, in welcher das Wasserstoffatom durch einen der oben charakterisierten Kohlenwasserstoffreste, vorzugsweise einen Niederalkyl- oder Phenylniederalkylrest, ersetzt ist; als Beispiel einer veresterten Carboxylgruppe sind insbesondere die Methoxy-, Ethoxy-, tert-Butoxy- und Benzyloxycarbonylgruppe, sowie auch eine lactonisierte Carboxylgruppe zu nennen.

Eine bevorzugte Aminogruppe ist eine solche der Formel

$$R^1-\overset{|}{N}-R^2 ,$$

worin $R^1$ und $R^2$ unabhängig je Wasserstoff, unsubstituiertes acyclisches $C_1$-$C_7$-Hydrocarbyl (wie insbesondere $C_1$-$C_4$-Alkyl oder $C_1$-$C_4$-Alkenyl) oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, wobei die kohlenstoffhaltigen Reste durch eine Kohlenstoff-Kohlenstoff-Bindung oder ein Sauerstoff-, Schwefel- oder gegebenenfalls durch Hydrocarbyl substituiertes Stickstoffatom untereinander gebunden sein können. In einem solchen Fall bilden sie zusammen mit dem Stickstoffatom der Aminogruppe einen stickstoffhaltigen heterocyclischen Ring.

Ein bevorzugter Hydrocarbylrest R° im Acylrest R°-C(=O)- ist z.B. $C_1$-$C_{19}$-Alkyl oder $C_1$-$C_{19}$-Alkenyl, insbesondere ein solcher, der bei mehr als 5 C-Atomen eine lineare Kette aufweist und welcher die folgenden Substituenten tragen kann: eine Carboxylgruppe, die gegebenenfalls auch in Salzform oder als eine Cyanogruppe, eine Carbamoylgruppe oder ein $C_1$-$C_4$-Alkylester ($C_1$-$C_4$-Alkoxycarbonylgruppe) vorliegen kann und welche sich vorzugsweise in ω-Stellung befindet, eine Aminogruppe der oben definierten Formel

$$R^1-\overset{|}{N}-R^2 ,$$

oder ein oder mehrere Halogenatome, insbesondere Fluor oder Chlor, die sich vorzugsweise in der Nachbarschaft der Carbonylgruppe befinden. Ein anderes bevorzugtes Acyl dieser Art ist ein bicyclisches oder insbesondere monocyclisches Aroyl, vor allem Benzoyl, welches auch einen oder mehrere der folgenden Substituenten tragen kann: Halogenatome, insbesondere Chlor oder Fluor, Nitrogruppen, $C_1$-$C_4$-Alkylreste, insbesondere Methyl, Hydroxylgruppen und veretherte Hydroxylgruppen, insbesondere $C_1$-$C_4$-Alkoxy, wie Methoxy, Phenoxy und Methylendioxy, sowie Carboxylgruppen, welche auch in der Salzform oder als eine Cyanogruppe oder ein $C_1$-$C_4$-Alkylester ($C_1$-$C_4$-Alkoxycarbonyl) vorliegen können. Vorzugsweise tragen die Aroylreste nicht mehr als 2, vor allem nur einen solchen Substituenten. Bevorzugt sind auch analoge Heteroaroylreste, insbesondere solche, die sich von Pyridin, Furan, Thiophen und Imidazol, und von ihren Analogen mit kondensiertem Benzoring (wie Chinolin, Isochinolin, Benzofuran und Benzimidazol) ableiten und gegebenenfalls auch, wie oben angegeben substituiert sind. Bevorzugte Acylreste dieser Art leiten sich auch vom Benzyl und Styryl ab (d.h. Phenacetyl und Cinnamoyl), sie können auch in der oben angegebenen Weise substituiert sein.

Carbonsäuren, die dem besonders bevorzugten Acylrest der Formel R°-C(=O)-zugrundeliegen, sind bespielsweise die folgenden: aliphatische Monocarbonsäuren mit höchstens 20 Kohlenstoffatomen, wie Niederalkancarbonsäuren, z.B. Propion-, Butter-, Isobutter-, Valerian-, Isovalerian, Capron-, Trimethylessig-, Oenanth- und Diethylessigsäure und vor allem Essigsäure, sowie Laurin-, Myristin-, Palmitin- und Stearinsäure, sowie Oelsäure, Elaidinsäure, Linolsäure und Linolensäure, aber auch entsprechende halogenierte Niederalkancarbonsäuren, wie die Trifluoressig-, Chloressigsäure, Bromessig- oder α-Bromisovaleriansäure, carbocyclische oder carbocyclisch-acyclische Monocarbonsäuren, z.B. die Cyclopropan-, Cyclopentan- und Cyclohexan-carbonsäure bzw. die Cyclopentan oder Cyclohexan-essigsäure oder -propionsäure; aromatische carbocyclische Carbonsäuren, z.B. Benzoesäure, die einfach oder mehrfach, wie oben angegeben, substituiert sein kann; Aryl- oder Aryloxy-niederalkancarbonsäuren und deren in der Kette ungesättigte Analoga, z.B. gegebenenfalls, wie oben für die Benzoesäure angegeben, substituierte Phenylessig- bzw. Phenoxyessigsäuren, Phenylpropionsäuren und Zimtsäuren; und heterocyclische Säuren, z.B. Furan-2-carbonsäure, 5-tert-Butylfuran-2-carbonsäure, Thiophen-2-carbonsäure, Nicotin- oder Isonicotinsäure, 4-Pyridinpropionsäure, und gegebenenfalls durch Niederalkylreste substituierte Pyrrol-2-oder -3-carbonsäuren; ferner auch entsprechende α-Aminosäuren, insbesondere die in der Natur vorkommenden α-Aminosäuren der L-Reihe, z.B. Glycin, Phenylglycin, Prolin, Leucin, Valin, Tyrosin, Histidin und Asparagin, vorzugsweise in einer N-geschützten Form, d.h. in einer solchen, in welcher die Aminogruppe durch eine konventionelle, z.B. eine der obengenannten, Aminoschutzgruppe substituiert ist; weiter auch Dicarbonsäuren, wie Oxalsäure, Malonsäure, Mono- oder Di-niederalkylmalonsäuren, Bernsteinsäure, Glutarsäure, Adipinsäure, Erucasäure, Maleinsäure, eine durch Halogen, wie Fluor, Chlor oder Brom, und/oder Niederalkyl, Hydroxy, Niederalkoxy und Nitro gegebenenfalls substituierte Phthal-, Chinolin-, Isochinolin- oder Phenylbernstein-säure, sowie auch Glutaminsäure und Asparaginsäure, wobei die zwei letztgenannten Säuren vorzugsweise mit geschützten Aminogruppen vorliegen. Wie bereits gesagt wurde, kann die zweite Carboxylgruppe nicht nur frei, sondern auch funktionell abgewandelt, z.B. als ein $C_1$-$C_4$-Alkylester, ein Amid oder ein Salz, vorzugsweise als ein physiologisch verträgliches Salz, mit einer salzbildenden basischen Komponente vorhanden sein. In Betracht kommen in erster Linie Me-

tall- oder Ammoniumsalze, wie Alkalimetall- und Erdalkalimetall-, z.B. Natrium-, Kalium-, Magnesium- oder Calciumsalze, bzw. Ammoniumsalze mit Ammoniak oder geeigneten organischen Aminen.

Ein von Monoestern der Kohlensäure abgeleiteter Acylrest Ac ist durch die Teilformel R°-O-CO- charakterisiert. Als Beispiel solcher Acylreste sind diejenigen zu nennen, worin R° die folgenden bevorzugten Bedeutungen eines acyclischen Hydrocarbylrestes darstellt: $C_1$-$C_{20}$-Alkyl, insbesondere $C_1$-$C_{10}$-Alkyl, $C_2$-$C_{20}$-Hydroxyalkyl, dessen Hydroxylgruppe sich in beliebiger Stellung ausser 1-Stellung, vorzugsweise in 2-Stellung, befindet, Cyano-[$C_1$-$C_{20}$]-alkyl, dessen Cyanogruppe sich vorzugsweise in 1-oder ω-Stellung befindet, oder vorzugsweise Carboxy-[$C_1$-$C_{20}$]-alkyl, insbesondere Carboxy-niederalkyl, dessen Carboxylgruppe sich vorzugsweise in 1- oder ω-Stellung befindet und gegebenenfalls auch in Salzform oder als Carbamoyl bzw. $C_1$-$C_4$-Alkylester ($C_1$-$C_4$-Alkoxycarbonyl) oder Benzylester (Benzyloxycarbonyl) vorliegen kann, sowie vor allem lineares (Mono-, Di- bis Hexa)-oxaalkyl mit 4-20, vorzugsweise 4-10 Kettengliedern, worin eines oder mehrere der C-Atome, von C-3 an, eines linearen $C_4$-$C_{20}$-Alkyls durch Sauerstoffatome, welche voneinander durch mindestens 2 C-Atome getrennt sind und sich vorzugsweise in den Stellungen 3, 6, 9, 12, 15 und 18 befinden, ersetzt sind. Bevorzugte Vertreter der letztgenannten Oxaalkylreste sind z.B. 3-Oxa-n-heptyl, 3,6-Di-oxa-n-octyl und 3,6-Di-oxa-n-decyl, wobei das Präfix "n" "normal" bedeutet, d.h. die geradkettigen Reste kennzeichnet.

Ein bevorzugter Vertreter der obengenannten veresterten Carboxy-[$C_1$-$C_{20}$]-alkylreste ist z.B. Ethoxycarbonylmethyl.

Salze von Verbindungen der obigen Formel I mit salzbildenden Eigenschaften leiten sich in erster Linie von denjenigen ab, worin B für Wasserstoff steht und/oder sich in einem Acylrest Ac und/oder im Rest der Formel II eine freie Aminogruppe als Substituent befindet, und sind Säureadditionssalze, insbesondere pharmazeutisch verwendbare, nichttoxische Säureadditionssalze mit anorganischen Säuren, z.B. Chlorwasserstoffsäure, Bromwasserstoffsäure, Schwefelsäure oder Phosphorsäuren, oder mit organischen Säuren, wie Sulfonsäuren, wie aromatischen Sulfonsäuren, z.B. Benzolsulfonsäure, p-Toluolsulfonsäure oder Naphthalin-2-sulfonsäure, oder insbesondere aliphatischen Sulfonsäuren, z.B. Methansulfonsäure, Ethansulfonsäure, 2-Hydroxyethansulfonsäure und Ethan-1,2-disulfonsäure, sowie auch Carbonsäuren, z.B. Essigsäure, Trifluoressigsäure, Propionsäure, Glykolsäure, Bernsteinsäure, Maleinsäure, Hydroxymaleinsäure, Benzoesäure, Zimtsäure, Mandelsäure, Salicylsäure, 4-Aminosalicylsäure, 2-Phenoxybenzoesäure, 2-Acetoxy-benzoesäure, Embonsäure, Nicotinsäure oder Isonicotinsäure.

Infolge der engen Beziehung zwischen den neuen Verbindungen in freier Form und in Form ihrer Salze, inkl. auch solcher Säureadditionssalze, die als Zwischenprodukte, z.B. bei der Reinigung der neuen Verbindungen oder zu ihrer Identifikation verwendet werden können, sind vorausgehend und nachfolgend unter den freien Verbindungen sinn- und zweckgemäss gegebenenfalls auch die entsprechenden Salze zu verstehen.

Die Verbindungen der vorliegenden Erfindung weisen wertvolle Eigenschaften, in erster Linie pharmakologische Wirkungen auf, indem sie eine physiologische Wirkung zeigen, die im Grundcharakter der Wirkung des Desferrioxamins B ähnlich ist. Sie können deshalb in ähnlichen therapeutischen Indikationen wie dieses, jedoch mit dem wesentlichen Vorteil der oralen oder rektalen Verabreichung, angewendet werden, z.B. insbesondere zur Behandlung von Funktionsstörungen, in denen die Konzentration des dreiwertigen Eisens ($Fe^{3+}$-Ions) in Körperzellen übernormal hoch ist, wie bei Hämochromatose und Hämosiderose. Indem sie überdies noch in ähnlicher Weise auch Aluminium-Ionen, wie z.B. bei Dialyse-Encephalopathie, Osteomalacia und der Alzheimer-Krankheit, binden, können sie auch in diesen Indikationsbereichen mit Erfolg eingesetzt werden.

Besonders wertvoll sind diejenigen Verbindungen der Formel I, worin $A^1$, $A^2$ und $A^3$ je ein und denselben Carbamoylrest der Teilformel II und B einen Acylrest bedeuten, welcher von Alkyl-O-($CH_2$-$CH_2$-O-)$_q$CO-, worin q eine Zahl grösser als 5 bedeutet, verschieden ist.

Hervorgehoben seien diejenigen Verbindungen der Formel I, worin B $C_2$-$C_{10}$-Alkanoyl, ($C_1$-$C_9$-Alkoxy)-carbonyl, Diniederalkylamino-carbonyl, lineares, Mono-, Di- oder Tri-oxa-alkoxy-carbonyl mit bis zu 18 Kettengliedern oder ω-($C_1$-$C_4$-Alkoxycarbonyl)-niederalkoxycarbonyl bedeutet.

Bevorzugt sind Verbindungen der Formel I, in welcher $A^1$, $A^2$ und $A^3$ die gleiche Bedeutung haben und jeweils für einen Carbamoylrest der Teilformel II stehen, worin $R^1_a$ $C_1$-$C_4$-Alkyl und Alk $C_1$-$C_5$-Alkylen bedeuten, und B für Wasserstoff, $C_2$-$C_{10}$-Alkanoyl, lineares Mono-, Di- oder Trioxa-alkoxycarbonyl mit bis zu 18 Kettengliedern oder ω-($C_1$-$C_4$-Alkoxycarbonyl)-niederalkoxycarbonyl stehen. Von diesen Verbindungen seien besonders diejenigen Verbindungen der Formel I hervorgehoben, in welcher $A^1$, $A^2$ und $A^3$ die gleiche Bedeutung haben und jeweils ω-Ethoxycarbonyl-($C_1$-$C_2$-alkylamino)-carbonyl bedeuten und B für 2-($C_2$-$C_4$-Alkoxy)-ethoxycarbonyl, 2-[2-($C_2$-$C_4$-Alkoxy)-ethoxy]-ethoxycarbonyl oder Ethoxycarbonylmethoxycarbonyl steht.

Bevorzugt sind insbesondere Verbindungen der Formel I, in welcher $A^1$, $A^2$ und $A^3$ die gleiche Bedeutung haben und jeweils für einen Carbamoylrest der Teilformel II stehen, worin $R^1_a$ $C_1$-$C_4$-Alkyl und Alk Methylen oder Ethylen bedeuten, und B für 2-($C_2$-$C_4$-Alkoxy)-ethoxycarbonyl, 2-[2-($C_2$-$C_4$-Alkoxy)-ethoxy]-ethoxycarbonyl,

Ethoxycarbonylmethoxycarbonyl oder ($C_1$-$C_4$-Alkoxy)-carbonyl steht.

Bevorzugt sind vor allem die in den Beispielen genannten Verbindungen der Formel I.

Erfindungsgemäss werden Verbindungen der Formel I oder Salze einer solchen Verbindung mit salzbildenden Eigenschaften unter Anwendung konventioneller Verfahren hergestellt, indem man

a) ein Derivat von Desferrioxamin B der Formel IV,

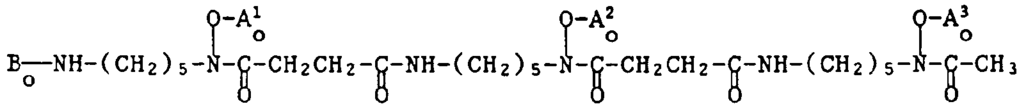

(IV)

worin $B_o$ ein Acylrest Ac, welcher von Alkyl-O-($CH_2$-$CH_2$-O-)$_q$CO-, worin q eine Zahl grösser als 5 bedeutet, verschieden ist, oder eine Aminoschutzgruppe ist und mindestens eines der Symbole $A_o^1$, $A_o^2$ und $A_o^3$ Wasserstoff bedeutet und die übrigen der Symbole $A_o^1$, $A_o^2$ und $A_o^3$ einen oben definierten Acylrest Ac bedeuten, mit einem Isocyanatocarbonsäureester der Formel III,

$$O=C=N\text{-Alk-CO-O-}R_a^1 \qquad \text{(III)}$$

worin $R_a^1$ und Alk die oben genannten Bedeutungen haben, wobei, falls im Rest Alk Amino- oder Hydroxygruppen vorhanden sind, diese durch leicht abspaltbare Schutzgruppen geschützt sind, umsetzt, und vorhandene Schutzgruppen abspaltet, oder

b) zur Herstellung einer Verbindung der Formel I, worin B für einen Acylrest steht, welcher von Alkyl-O-($CH_2$-$CH_2$-O)$_q$CO-, worin q eine Zahl grösser als 5 bedeutet, verschieden ist, eine Verbindung der Formel I, worin B für Wasserstoff oder eine Silylgruppe steht, acyliert, und, wenn erwünscht, nach Durchführung der Verfahren a) oder b) eine erhaltene freie Verbindung der Formel I mit salzbildenden Eigenschaften in eines ihrer Salze umwandelt oder eine Verbindung der Formel I aus einem ihrer Salze freisetzt.

Die erfindungsgemässen Verfahren und die Herstellung der Ausgangsstoffe werden im folgenden näher erläutert:

Verfahren a)

Die Erfindung betrifft auch diejenigen Ausführungsformen des Verfahrens, worin man anstelle des Isocyanatocarbonsäureesters der Formel III ein anderes reaktionsfähiges Carbaminsäurederivat verwendet, z.B. das bei der Herstellung des Isocyanatocarbonsäureesters als Zwischenprodukt anfallende Säurechlorid der Formel Cl-C(=O)-NH-Alk-CO-O-$R_a^1$. Man kann auch reaktionsfähige Carbaminsäureester verwenden, z.B. erhält man einen Ester der Formel $H_3C$-$CH_2$-O-C(=O)-NH-Alk-CO-O-$R_a^1$ aus dem Aminosäureester mit Diethylcarbonat in Gegenwart katalytischer Mengen Natriummethylat.

Als Schutzgruppen werden im Rahmen dieses Textes nur solche Gruppen bezeichnet, die nicht Bestandteil des gewünschten spezifischen Endstoffes der Formel I sind und die daher an geeigneter Stelle des Herstellungsverfahrens wieder abgespalten werden.

Zum vorübergehenden Schutz einer gegebenenfalls vorhandenen Aminogruppe während der erfindungsgemässen Umsetzung eignen sich die üblichen Aminoschutzgruppen, welche bei der Synthese der Peptidkette verwendet werden und einschliesslich entsprechender Abspaltungsmethoden in Uebersichtsreferaten und Nachschlagewerken, wie Houben-Weyl: Methoden der organischen Chemie; 4. Auflage, Band 15/I und II, E. Wünsch (Herausgeber): Synthese von Peptiden (Georg Thieme Verlag, Stuttgart; 1974), ausführlich beschrieben sind. Vorzugsweise werden acidolytisch oder neutral abspaltbare Aminoschutzgruppen verwendet.

Unter geeigneten Aminoschutzgruppen kommt z.B. durch Methyl, Methoxy, Halogene und/oder Nitro substituiertes Trityl in Betracht, oder vorzugsweise die unsubstituierte Trityl- (Triphenylmethyl-) Gruppe, welche unter sehr milden Bedingungen, wie bereits mit ca. 50%iger Essigsäure, solvolytisch (acidolytisch) abspaltbar ist. - Auch im Ring substituierte Phenylsulfenylgruppen, vor allem die 2-Nitrophenylsulfenylgruppe o-$O_2N$-$C_6H_4$-S-, sind zu erwähnen; die letztgenannte ist z.B. durch eine säurekatalysierte Solvolyse oder Acidolyse, z.B. bereits mit Pyridinhydrochlorid, abspaltbar.

Als wichtigste Aminoschutzgruppen kommen jedoch veresterte Oxycarbonylreste der Teilformel $R_o$-O-CO- in Frage, worin $R_o$ ein neutral und/oder acidolytisch abspaltbarer Hydrocarbylrest ist.

Derartige Aminoschutzgruppen sind beispielsweise gegebenenfalls im aromatischen Ring durch Halogenatome, Nitrogruppen, Niederalkyl-oder Niederalkoxygruppen substituierte Benzyloxycarbonylgruppen, wie

unsubstituiertes Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl und p-Tolyloxycarbonyl, bzw. Furfuryloxycarbonyl, sowie auch 2-(4-Biphenylyl)-2-propyloxycarbonyl und ähnliche im Schweizer Patent 509 266 beschriebene Aralkoxycarbonylreste. Diese Reste lassen sich, wie noch weiter unten näher beschrieben wird, unter neutralen Bedingungen hydrogenolytisch, oder aber vorzugsweise acidolytisch abspalten.

Ein weiterer solcher Acylrest $R_o$-O-CO- ist beispielsweise vor allem tert.-Butoxycarbonyl, oder auch ein analoger Rest, wie Isopropyloxycarbonyl, tert.-Amyloxycarbonyl (d.h. 1,1-Dimethylpropyloxycarbonyl), Diisopropylmethoxycarbonyl, Cyclopentyloxycarbonyl, Cyclohexyloxycarbonyl, d-Isobornyloxycarbonyl und Adamantyloxycarbonyl. Diese Reste lassen sich, wie noch weiter unten näher beschrieben wird, vornehmlich unter sauren Bedingungen (acidolytisch) abspalten.

Ein noch weiterer Acylrest $R_o$-O-CO-, ist z.B. ein β-(Trihydrocarbylsilyl)-ethoxycarbonylrest, wie β-(Triniederalkylsilyl)-ethoxycarbonyl, z.B. insbesondere β-(Trimethylsilyl)-ethoxycarbonyl. Solche Reste bilden mit der zu schützenden Aminogruppen entsprechende β-Trihydrocarbylsilylethoxycarbonylaminogruppen (z.B. die β-Trimethylsilylethoxycarbonylaminogruppe), welche unter den Bedingungen der sauren Hydrolyse und der Hydrogenolyse zwar beständig sind, aber unter ganz spezifischen, sehr milden Bedingungen durch Einwirkung von Fluoridionen sich abspalten lassen.

Besonders hervorzuheben ist auch Allyloxycarbonyl, welches sich nicht zur acidolytisch, sondern besonders auch unter sehr milden neutralen Bedingungen mit Dimedon, oder durch die spezifische reduktive Einwirkung von Tributylzinnhydrid unter Katalyse mit Palladium-(O)-tetrakis-(triphenylphosphin)-Komplex abspalten lässt.

Die erfindungsgemässe nachträgliche Abspaltung der Aminoschutzgruppe erfolgt in der allgemein bekannten Weise, wobei spezifische Bedingungen für einzelne Strukturtypen in der einschlägigen Literatur (siehe z.B. Houben-Weyl, loc. cit.) bis ins Detail beschrieben sind. Die Acidolyse (einschliesslich saurer Hydrolyse) wird z.B. mit Trifluoressigsäure, Fluorwasserstoff, Bromwasserstoff und Chlorwasserstoff, gegebenenfalls in Anwesenheit von Wasser, wie mit Salzsäure, und bei säureempfindlichen Schutzgruppen auch mit einer niederaliphatischen Carbonsäure, wie Ameisensäure und/oder Essigsäure, gegebenenfalls in Anwesenheit von Wasser durchgeführt. Die neutral abspaltbaren Gruppen, insbesondere solche, die Benzylreste enthalten, werden vorzugsweise hydrogenolytisch, z.B. durch Hydrierung unter Palladium-Katalyse, entfernt. Die β-Silylethoxycarbonylgruppen werden vorzugsweise mit Fluoridionen-abgebenden Mitteln, z.B. mit Fluoriden quaternärer organischer Basen, wie Tetraethylammoniumfluorid oder Tetrabutylammoniumfluorid, in neutralen organischen Lösungsmitteln abgespalten.

Die zum vorübergehenden Schutz von Hydroxylgruppen anzuwendenden Gruppen und Abspaltungsmethoden sind auch allgemein bekannt, z.B. aus der Synthese von Peptiden. Insbesondere schützt man Hydroxylgruppen in der Form von Estern mit Carbonsäuren, wie mit Niederalkansäuren oder mit Monoestern der Kohlensäure (z.B. Formiate oder Acetate einerseits oder tert-Butoxy- oder Benzyloxy-carbonate andererseits), oder aber in der Form von Ethern, wie insbesondere solchen von tertiären Alkoholen (z.B. tert-Butylalkohol), oder auch in der Form von Acetalen (z.B. insbesondere als 2-Tetrahydropyranylether). Die ersteren Schutzgruppen werden üblicherweise analog wie veresterte Carbonylgruppen abgespalten; beide letzteren werden vornehmlich durch Acidolyse entfernt.

Durch geeignete Wahl der Reaktionsbedingungen kann man die Reaktion so steuern, dass alle im Ausgangsstoff der Formel IV vorhandenen freien Hydroxamsäuregruppen substituiert werden. Zu diesem Zweck kann man beispielsweise einen Ueberschuss an Isocyanatocarbonsäureester der Formel III unverdünnt, oder aber gelöst in einem inerten Lösungsmittel, wie einem chlorierten Kohlenwasserstoff (z.B. Chloroform oder Dichlormethan) oder einem aromatischen Kohlenwasserstoff, z.B. Toluol, einsetzen.

Zur Herstellung von Verbindungen der Formel I, worin einer oder zwei der Reste $A^1$, $A^2$ und $A^3$ Wasserstoff bedeuten, kann man z.B. die Menge des Isocyanats der Formel III reduzieren und/oder die Reaktion früher abbrechen. Dabei entstehen Gemische von Verbindungen der Formel I, die, z.B. chromatographisch, aufgetrennt werden.

Zur Herstellung einer Verbindunge der Formel I, worin einer oder zwei der Reste $A^1$, $A^2$ und $A^3$ einen Acylrest bedeuten, kann man z.B. eine Verbindung der Formel I, worin einer oder zwei der Reste $A^1$, $A^2$ und $A^3$ Wasserstoff bedeuten, acylieren.

Zur Herstellung von Verbindungen der Formel I, worin B Wasserstoff bedeutet, geht man von einer Verbindung der Formel IV aus, worin $B_o$ eine Aminoschutzgruppe bedeutet, und spaltet diese nach beendeter Reaktion ab. Eine zu diesem Zweck besonders geeignetet Aminoschutzgruppe ist z.B. die tert. Butoxycarbonylgruppe, die z.B. in einem Toluol-Anisol-Gemisch bei 2-10°C mit Trifluoressigsäure abgespalten werden kann.

Die Umsetzung mit dem Isocyanat der Formel III wird unter strengem Ausschluss von Wasser und protischer Lösungsmittel (wie insbesondere von Niederalkanolen) durchgeführt. Die Reaktionstemperatur liegt übli-

cherweise zwischen etwa 0 bis etwa 80°C, vornehmlich in der Umgebung der Raumtemperatur oder leicht oberhalb davon. Die Umsetzung erfolgt unter basischen Bedingungen, z.B. in Pyridin unter Zusatz von etwas Triethylamin, und kann vornehmlich durch starke organische Basen, wie insbesondere 1,8-Diazabicyclo[5.4.0]undec-7-en und ähnliche cyclische Basen, oder 4-Dialkylaminopyridine, z.B. 4-Dimethylamino- oder 4-Diethylaminopyridin, katalytisch beschleunigt werden.

Ueblicherweise wird die Umsetzung in Lösung oder Suspension in aprotischen inerten organischen Lösungsmitteln oder ihren zweckmässigen Gemischen, wie cyclischen Ethern (z.B. Dioxan oder Tetrahydrofuran), tertiären Amiden (z.B. N,N-Dimethylformamid, N,N-Dimethylacetamid, N-Methylpyrrolidon und Hexamethylphosphortriamid), Dimethylsulfoxid, oder vorzugsweise in tertiären Aminen, wie Triethylamin, Ethyldiisopropylamin, Tributylamin, N,N-Dimethyl- und N,N-Diethylanilin, N-Methyl- und N-Ethylpiperidin oder -morpholin und N,N'-Dimethylpiperazin, sowie stickstoffhaltigen heteroaromatischen Basen, z.B. Pyridin, Collidin und Chinolin, oder in Acetonitril oder ähnlichen Niederalkylcyaniden durchgeführt. Zum besseren Vermischen von spärlich löslichen oder miteinander mischbaren Komponenten arbeitet man vorzugsweise unter intensivem Rühren.

Die Ausgangsstoffe der Formel IV, worin $A_o^1$, $A_o^2$ und $A_o^3$ für Wasserstoff stehen, kann man vorteilhaft herstellen, indem man von einer Verbindung der Formel IV ausgeht, worin $B_o$ und jedes der Symbole $A_o^1$, $A_o^2$ und $A_o^3$ für eine organische Silylgruppe (Sil) der Formel

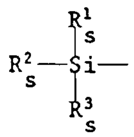 (Sil),

stehen, in welcher $R_s^1$ und $R_s^2$ unabhängig je unsubstituiertes $C_1$-$C_8$-Hydrocarbyl und $R_s^3$ unsubstituiertes $C_1$-$C_8$-Hydrocarbyl oder Chlor bedeutet.

Die in der organischen Silylgruppen Sil vorhandenen Hydrocarbylreste $R_s^1$ und $R_s^2$ sind insbesondere $C_1$-$C_8$-Alkylreste, beispielsweise Hexyl, 4-Methylpentyl, Pentyl, Ethyl und vor allem Methyl, ferner auch Aryl- und Aralkylreste, beispielsweise Phenyl oder p-Tolyl, bzw. Benzyl oder Phenethyl; vorzugsweise sind beide Reste gleich. Das Symbol $R_s^3$ kann für Chlor stehen oder eine der genannten Bedeutungen der Symbole $R_s^1$ und $R_s^2$ haben, wobei alle 3 Symbole vorzugsweise dieselbe Bedeutung haben; und vor allem bedeutet $R_s^3$ Methyl.

Eine geeignete organische Silylgruppe Sil ist beispielsweise Trimethylsilyl, Tribenzoylsilyl, Phenyldimethylsilyl, Benzyl-dimethylsilyl, Hexyl-dimethylsilyl, tert-Butyl-dimethylsilyl, Triethylsilyl, Diethylchlorsilyl und insbesondere Dimethyl-chlorsilyl und vor allem Trimethylsilyl.

Die N-Acylierung eines solchen N- und O-silylierten Ausgangsstoffes wird unter den für die Acylierung von Aminogruppen bekannten Bedingungen durchgeführt, wobei nur die N-silylierte Aminogruppe acyliert wird, wogegen die O-gebundenen Silylgruppen bestehen bleiben und erst in der anschliessenden Solvolyse unter Freisetzung der Hydroxamsäuregruppen entfernt werden. Die Solvolyse kann in konventioneller Weise mit einem protischen Reagens (einschliesslich Wasser), welches zugleich als Lösungsmittel dienen kann, und vorzugsweise unter Säurekatalyse durchgeführt werden. Vorteilhafterweise wird dem Reaktionsgemisch nach der N-Acylierung ein Niederalkanol, wie Ethanol oder vor allem Methanol, (und gegebenenfalls eine starke Säure, wie Chlorwasserstoff) zugesetzt, wodurch die abgespaltenen Silylgruppen zu leicht flüchtigen, abdestillierbaren niederaliphatischen Silylethern umgewandelt werden.

Vornehmlich kann man die oben beschriebenen N- und O-silylierten Ausgangsstoffe für die N-Acylierung direkt im Reaktionsmedium in situ bilden, indem man Desferrioxamin B oder ein Säureadditionssalz davon (bzw. ein teilweise O-acyliertes Analoges) in Gegenwart einer aprotischen organischen Base, wie einer oben genannten, vor allem Pyridin, (welche zugleich als Lösungsmittel dienen kann) mit einem Silylierungsreagens, insbesondere einem Silylhalogenid der Formel Sil-Hal, worin Sil die oben genannte Bedeutung hat und Hal für Brom oder insbesondere Chlor steht, umsetzt. Ein besonders bevorzugtes Silylierungsreagens ist z.B. ein Triniederalkylsilylchlorid, wie Trimethylsilylchlorid, oder auch ein Diniederalkyldichlorsilan, wie Dimethyldichlorsilan. Vornehmlich wird das Silylierungsmittel in überschüssiger Menge zugesetzt; seine Gegenwart stört oder beeinträchtigt die nachfolgende N-Acylierung in keiner Weise, im Gegenteil, sie entfernt jede mögliche Spur von störender Feuchtigkeit. Deshalb kann die N-Acylierung im Anschluss an die Silylierung im gleichen Reaktionsmedium vorgenommen werden und sogar mit der nachfolgenden solvolytischen Abspaltung der Silylgruppen zusammengelegt werden, so dass alle 3 Stufen (Herstellung des N- und O-silylierten Ausgangsstoffes, N-Acylierung und Abspalten der O-Silylgruppen) in ein und demselben Reaktionsmedium durchgeführt

werden können. Das überschüssige Silylierungsreagens, sowie auch das überschüssige Acylierungsmittel, werden dabei vorteilhaft unter den Bedingungen der Solvolyse zerstört und in flüchtige Produkte umgewandelt; die Silylhalogenide liefern dabei Chlorwasserstoff, der die Solvolyse vorteilhaft katalysiert.

Die als Ausgangstoffe verwendeten Isocyanatocarbonsäureester der Formel III und die anderen reaktionsfähigen Carbaminsäurederivate sind bekannt oder können in an sich bekannter konventioneller Weise, z.B. durch Behandeln eines Aminosäureesters in Form eines Säureadditionssalzes davon, und gegebenenfalls unter Schutz einer als Substituent vorhandenen, nicht umzusetzenden Amino- oder Hydroxygruppe mit einer mindestens äquivalenten, vorzugsweise überschüssigen Menge Phosgen, gegebenenfalls in Anwesenheit eines nicht-acylierbaren Amins (wie eines oben genannten) erhalten werden. Erhaltene Verbindungen der Formel III mit geschützter Amino- oder Hydroxygruppe werden vorteilhaft als solche in der Hauptreaktion eingesetzt und die Schutzgruppe erst nachträglich abgespalten.

Verfahren b)

Als einen Acylrest Ac einführende Mittel werden die üblichen, zu diesem Zweck allgemein gebräuchlichen Acylierungsmittel verwendet; insbesondere verwendet man Acylierungsmittel der Formel AcY, worin Ac die oben angegebenen allgemeinen und hervorgehobenen Bedeutungen hat, und Y eine reaktionsfähige funktionell abgewandelte Hydroxylgruppe ist oder eine zusätzliche einfache Bindung zum Rest Ac darstellt, deren anderes Ende ein Wasserstoffatom im Rest Ac ersetzt.

Ein Acylierungsmittel, das sich vom oben definierten Acylrest Ac ableitet, ist insbesondere ein solches, worin Y eine veresterte Hydroxylgruppe ist, beispielsweise eine solche, die mit einer starken anorganischen Säure, wie einer Halogenwasserstoffsäure, (z.B. Chlor-, Brom-oder Iodwasserstoffsäure), einer Pseudohalogenwasserstoffsäure, wie Azoimid oder Imidazol (unter Abspaltung des H-Atoms von 1-N-Atom), einer sauerstoffhaltigen Mineralsäure, wie Phosphorsäure und insbesondere Schwefelsäure, oder einer starken organischen, wie aliphatischen oder aromatischen Sulfonsäure, (z.B. Methan- und Ethan- bzw. Benzol-, p-Toluol-, p-Nitrobenzol- und p-Chlorbenzolsulfonsäure) verestert wird. Eine derartige veresterte Gruppe bildet dann mit dem Acylrest ein gemischtes Anhydrid. Darunter besonders hervorzuheben sind gemischte Anhydride mit Halogenwasserstoffsäuren oder Pseudohalogenwasserstoffsäuren, wie Säurebromide, Säurechloride, Säureazide und 1-Imidazolyl-Derivate der Formel R°-CO-Hal bzw. R°-O-CO-Hal, worin Hal Brom oder Azido oder vorzugsweise Chlor oder 1-Imidazolyl bedeutet und R° die oben genannten Bedeutungen hat. Als ein Reagens dieses Typs, welches insbesondere bei Herstellung von Ausgangsstoffen für die Verfahrensvariante b) von Wichtigkeit ist, sind Phosgen und sein weniger toxisches Analogon Bis-(1-imidazolyl)-carbonyl (und ähnliche Reagentien) zu erwähnen. Diese setzt man in der Regel in äquimolaren Mengen ein, so dass die zweite reaktionsfähige Gruppe Y im Produkt erhalten bleibt und nachträglich abgewandelt werden kann.

Als Beispiel für ein Acylierungsmittel AcY zur Einführung eines Diniederalkylamino-carbonylrestes, z.B. des Diethylamino-carbonylrestes, sei Diniederalkyl-carbamoylchlorid, z.B. Diethylcarbamoylchlorid, genannt.

Die reaktionsfähige veresterte Hydroxylgruppe kann aber auch entweder durch den Rest einer anderen Carbonsäure, insbesondere einer stärkeren Carbonsäure, wie der Ameisensäure, Chloressigsäure oder vornehmlich der Trifluoressigsäure, verestert werden und einem gemischten Anhydrid zugrundeliegen, oder aber durch denselben Acylrest verestert werden und ein symmetrisches Carbonsäureanhydrid der Formel $Ac^1$-O-$Ac^1$, insbesondere eines der Formeln R°-CO-O-CO-R° oder R°-O-CO-O-CO-O-R° bilden.

Acylierungsmittel der Formel AcY, in welchen Y eine zusätzliche Bindung zum Rest Ac darstellt, leiten sich insbesondere von Acylresten der Carbonsäuren ab, die am C-Atom, das der Carboxylgruppe benachbart ist, Wasserstoff tragen; sie gehören der Kategorie der Ketene der Formel $R^\circ_a$=C=O, worin $R^\circ_a$ für Hydrocarbyliden steht, d.h. für einen zweiwertigen, dem Rest R° entsprechenden Rest aliphatischen Charakters, in welchem das funktionalisierte Kohlenstoffatom durch einfache Bindungen mit benachbarten Kohlenstoff- und/oder Wasserstoffatomen verbunden ist.

Die Umsetzung mit dem Acylierungsmittel der Formel AcY erfolgt unter bekannten Verfahrensbedingungen, die in der organischen Chemie allgemein für die Acylierung von Aminen gebräuchlich sind, üblicherweise bei Temperaturen zwischen dem Gefrierpunkt und dem Siedepunkt des Reaktionsgemisches, wie im Temperaturbereich von etwa -10 bis etwa +160°, insbesondere von etwa +20 bis etwa +50°, beim atmosphärischen oder erhöhten Druck, in heterogener Phase (wie Suspension) unter Rühren oder Umschütteln, oder vornehmlich in homogener flüssiger Phase, wie in einem Ueberschuss von flüssigem Reagens oder insbesondere in Anwesenheit von Lösungsmitteln, insbesondere organischen Lösungsmitteln, und gegebenenfalls in Gegenwart von säurebindenden anorganischen oder organischen Mitteln. Geeignete Lösungsmittel sind beispielsweise aprotische organische Lösungsmittel niedriger Polarität, wie halogenierte, insbesondere chlorierte, aliphatische Kohlenwasserstoffe, wie Chloroform und Dichlormethan, und insbesondere polare aprotische

Lösungsmittel, wie aliphatische und cyclische Ether, z.B. Diethylether, 1,2-Dimethoxyethan und Diisopropylether bzw. Dioxan und Tetrahydrofuran, niederaliphatische Ester und Amide, wie Ethylacetat bzw. Formamid, Acetamid, N,N-Dimethylacetamid und Dimethylformamid, sowie Acetonitril, Dimethylsulfoxid und Hexamethylphosphortriamid; die Lösungsmittel können auch in zweckmässigen Kombinationen, z.B. zur Erhöhung der Löslichkeit von Komponenten, eingesetzt werden.

Als säurebindende Mittel können im Prinzip beliebige basische Verbindungen zugezogen werden, wie einerseits organische stickstoffhaltige Basen, z.B. tertiäre Amine vom Typ Triethylamin, Ethyldiisopropylamin, N,N-Dimethylanilin, N-Ethylpiperidin oder N,N′-Dimethylpiperazin, oder aromatische heterocyclische Basen vom Typ Pyridin, Collidin, Chinolin oder 4-Dimethylaminopyridin, andererseits basisch reagierende anorganische Verbindungen, wie Alkalimetallhydroxide, -carbonate und -hydrocarbonate, sowie Salze von Carbonsäuren, wie Natrium- oder Kaliumacetat. Schliesslich können, diese Rolle auch neutral reagierende stickstoffhaltige Verbindungen übernehmen, die zugleich oft auch vorteilhafte Lösungsmittel darstellen, z.B. Carbonsäureamide, insbesondere niederaliphatische Carbonsäureamide, wie die oben genannten, und cyclische Amide, wie N-Methylpyrrolidon, sowie Amidoderivate der Kohlensäure, wie Urethane und Harnstoff. Umgekehrt können die oben erwähnten Basen, insbesondere die vom Typ Pyridin, als Lösungsmittel dienen.

Falls der Hydrocarbylrest $R^{\circ}$ durch funktionelle Gruppen substituiert ist, die während der Acylierung mitreagieren könnten, wie freie Carboxyl-, Hydroxyl- und insbesondere Aminogruppen, werden diese vornehmlich vorübergehend geschützt, oder vorzugsweise im angewendeten Acylierungsmittel bereits in geschützter Form vorliegen, und nach erfolgter Acylierung von diesen Schutzgruppen befreit.

So z.B. gehört zu den gewöhnlichsten Methoden zum Schutz von Carboxylgruppen die Veresterung. Die Freisetzung einer veresterten Carboxylgruppe erfolgt im allgemeinen durch konventionelle Hydrolyse, vor allem unter Einwirkung von Basen (wie vornehmlich von Alkalimetall-hydroxiden, -carbonaten der -hydrocarbonaten), oder aber, bei geeigneten Estern, wie solchen von tertiären Alkoholen (z.B. tert-Butylalkohol), durch Acidolyse, z.B. mittels Fluorwasserstoff oder Trifluoressigsäure. Ester mit Benzylalkoholen können auch durch konventionelle Hydrogenolyse abgespalten werden.

Die zum vorübergehenden Schutz von Hydroxygruppen, sowie von primären und sekundären Aminogruppen verwendbaren Schutzgruppen entsprechen denjenigen, die oben eingehend diskutiert wurden.

Die Ausgangsstoffe für Verfahren b) erhält man durch Umsetzung einer Verbindung der Formel IV, worin $B_o$ für eine Aminoschutzgruppe steht, gemäss Verfahren a).

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung sind ebenfalls Gegenstand der vorliegenden Erfindung.

Die Erfindung betrifft auch diejenigen Ausführungsformen der Verfahren, bei denen ein Ausgangsstoff in Form eines reaktionsfähigen Derivats oder eines Salzes verwendet wird.

Die vorliegende Erfindung betrifft ebenfalls pharmazeutische Zusammensetzungen zur parenteralen oder enteralen Applikation, die als Wirkstoff eine der neuen pharmakologisch wirksamen Verbindungen der Formel I, insbesondere eine der oben für diese Anwendung hervorgehobenen, enthalten. Besonders bevorzugt sind Präparate und Zusammensetzungen zur enteralen, wie insbesondere oralen Verabreichung. Da die Resorption des Wirkstoffs nach oraler Applikation hauptsächlich aus dem Darm, insbesondere aus dem Dünndarm, erfolgt, werden solche oralen Darreichungsformen bevorzugt, die mit einem Magensaft-resistenten Ueberzug versehen sind. Die Präparate enthalten den Wirkstoff allein oder vorzugsweise zusammen mit einem pharmazeutisch anwendbaren Trägermaterial, insbesondere in einer Menge, die zur Behandlung von krankhaften Zuständen in Warmblütern einschliesslich des Menschen, welche mit einem Ueberschuss an Eisen(III) oder Aluminium im Körper zusammenhängen oder durch Eisen(III)-abhängige pathogene Organismen verursacht werden, wirksam ist. Die Dosierung des Wirkstoffs hängt von der zu behandelnden Krankheit, sowie von der Spezies, deren Alter, Gewicht und individuellem Zustand, sowie von der Applikationsweise ab, im allgemeinen entspricht sie jedoch auf molarer Basis mengenmässig etwa derjenigen für parenteral appliziertes Desferrioxamin B oder einem Salz davon.

Die pharmazeutischen Zusammensetzungen enthalten vorzugsweise von etwa 5 % bis etwa 95 % des Wirkstoffs, wobei einzeldosierte Applikationsformen vorzugsweise von etwa 20 % bis etwa 90 % und nichteinzeldosierte Applikationsformen vorzugsweise etwa 5 % bis etwa 20 % Wirkstoff aufweisen; pharmazeutische Präparate in Dosiseinheitsform, wie Dragées, Tabletten oder Kapseln, bzw. Suppositorien enthalten von etwa 0,1 g bis etwa 3,0 g, vorzugsweise von etwa 0,3 g bis etwa 1,0 g des Wirkstoffs.

Die pharmazeutischen Zusammensetzungen der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen

Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol.

Dragée-Kerne werden mit geeigneten, vorzugsweise Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Zusammensetzungen sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten oder wachsartigen Substanzen, wie fetten Oelen, Paraffinöl oder Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Die Erfindung betrifft ebenfalls ein Verfahren zur Behandlung von Krankheiten, bei denen, wie oben beschrieben worden ist, ein Ueberschuss von Eisen(III) oder Aluminium im Körper vorhanden ist, dadurch gekennzeichnet, dass man eine prophylaktisch oder therapeutisch wirksame Menge, insbesondere eine zur Behandlung von krankhaften Zuständen in Warmblütern einschliesslich des Menschen, welche mit einem Ueberschuss an Eisen(III) oder Aluminium im Körper zusammenhängen oder durch Eisen(III)-abhängige pathogene Organismen verursacht werden, wirksame Menge einer Verbindung der Formel I, vorzugsweise peroral, verabreicht. Dabei verwendet man in erster Linie die obengenannten pharmazeutischen Zusammensetzungen, wobei man einem Warmblüter von etwa 70 kg Gewicht eine tägliche Dosis von etwa 0,5 g bis etwa 15 g, vorzugsweise von etwa 1,5 g bis etwa 7,5 g einer Verbindung der vorliegenden Erfindung verabreicht.

Die nachfolgenden Beispiele illustrieren die vorliegende Erfindung; Temperaturen werden in Celsiusgraden angegeben.

Die säulenchromatographische Reinigung von Verbindungen der Formel I wird, wenn nicht anders angegeben, in Dichlormethan-Isopropanol mit einem Gehalt von bis zu 5 Volumenprozent Isopropanol durchgeführt. Die Fraktionengrösse beträgt, wenn nicht anders angegeben, 150 ml.

Die $R_f$-Werte werden, wenn nicht anders angegeben, auf Kieselgel Dünnschichtplatten in den folgenden Lösungsmittelsystemen (v/v) ermittelt:

(1): Methylenchlorid-Aceton (7:3)
(2): Methylenchlorid-Isopropanol (9:1)
(3): Methylenchlorid-Methanol (9:1)
(4): Methylenchlorid-Methanol (4:1)

Zum Beispiel bedeutet $R_f(1)$ ein im System (1) ermittelter $R_f$-Wert.

Die Hochdruckflüssigkeitschromatographie wird, wenn nicht anders angegeben, in folgendem System durchgeführt:

Säule: Hypersil ODS (ein mit $C_{18}$-Kohlenwasserstoffketten modifizierter, sphärischer, poröser Silikagelträger), Korngrösse (Durchmesser) 5 µm, Länge 120 mm, Durchmesser 4,6 mm, Lösung A = 2,5 millimolarer Phosphatpuffer pH 3,0, Lösung B = 20 % Lösung A und 80 % Acetonitril, Gradient:

| Minuten | % A | % B | Fluss [ml/Minuten] |
|---|---|---|---|
| 0 | 100 | 0 | 2,3 |
| 12 | 0 | 100 | 2,3 |
| 14 | 100 | 0 | 2,3 |
| 15 | 100 | 0 | 2,3 |

Die durch Hochdruckflüssigkeitschromatographie erhaltenen $R_f$-Werte werden im folgenden als $R_f$(HPLC) bezeichnet.

In den nachfolgenden Beispielen werden die Umsetzungen mit den verschiedenen Isocyanaten und die Herstellung der Isocyanate unter Feuchtigkeitsausschluss, d.h. unter absoluten Bedingungen durchgeführt.

Beispiel 1:

Zu 7,04 g (10 mMol) N-(2-Butoxy-ethoxycarbonyl)-desferrioxamin B in 500 ml Pyridin gibt man zunächst 2,8 ml (20 mMol) Triethylamin und dann 4,5 ml (40 mMol) Isocyanatoessigsäureethylester und rührt 1,5 Stunden bei Raumtemperatur. Man desaktiviert das überschüssige Reagenz mit Methanol, dampft ein, löst den Rückstand in 300 ml Methylenchlorid, wäscht mehrmals mit Wasser und chromatographiert über eine Säule mit Kieselgel (15-25 μm). Man erhält aus den Fraktionen 5-11 N-(2-Butoxyethoxy-carbonyl)-O,O′,O″-tri-(ethoxycarbonylmethylcarbamoyl)-desferrioxamin B als farbloses hygroskopisches Harz; $R_f(1)$ = 0,10, $R_f(2)$ = 0,70, $R_f(3)$ = 0,85, $R_f(4)$ = 0,90, $R_f$(HPLC) = 8,80 Minuten (Edukt: 6,71 Minuten).

Aus den Fraktionen 12-17 erhält man die entsprechende Verbindung, die jedoch nur zwei, statt 3 Carbamoylgruppen trägt.

Das Ausgangsprodukt erhält man folgendermassen:

Stufe 1.1:

19,6 ml (150 mMol) 2-Butyloxyethanol in 300 ml Toluol werden mit 75 ml (150 mMol) 20%igem Phosgen in Toluol versetzt und 3 Stunden bei 100°C gerührt. Die Reaktionslösung, die Chlorameisensäure-(2-butyloxyethyl)-ester, enthält, wird abgekühlt und in Stufe 1.2 weiterverwendet.

Stufe 1.2:

Zu 45,92 g (70 mMol) Desferrioxamin B-methansulfonat in 800 ml Pyridin werden 106 ml (840 mMol) Trimethylchlorsilan gegeben. Nach zweistündigem Rühren bei Raumtemperatur tropft man zu der erhaltenen Reaktionslösung, welche N,O,O′,O″-Tetra-(trimethylsilyl)-desferrioxamin B enthält, 240 ml (90 mMol) der gemäss Stufe 1.1 erhaltenen Reaktionslösung innerhalb von 30 Minuten. Nach zweistündigem Rühren bei Raumtemperatur desaktiviert man das überschüssige Reagenz durch Zugabe von 400 ml Methanol und dampft ein. Der Rückstand wird aus 500 ml Wasser kristallisiert. Die Kristalle werden in Essigsäureethylester aufgenommen, verrührt und filtriert. Man erhält N-(2-Butoxy-ethoxy-carbonyl)-desferrioxamin B; Smp. 143-144°C.

Beispiel 2:

Analog Beispiel 1 erhält man aus 7,04 g (10 mMol) N-(2-Butoxy-ethoxy-carbonyl)-desferrioxamin B (s. Stufe 1.2) und 120 ml (40 mMol) des 3-Isocyanato-propionsäure-ethylester enthaltenden Reaktionsgemisches aus Stufe 2.1 N-(2-Butoxyethoxy-carbonyl)-O,O′,O″-tri-(N-[2-ethoxycarbonyl-ethyl]-carbamoyl)-desferrioxamin B als hygroskopisches öliges Harz; $R_f(1)$ = 0,10, $R_f(2)$ = 0,70, $R_f(3)$ = 0,80, $R_f(4)$ = 0,95, $R_f$(HPLC) = 9,02 Minuten (Edukt: 6,71 Minuten).

Aus den Fraktionen 13-17 erhält man die entsprechende Verbindung, die jedoch nur zwei, statt 3 Carbamoylgruppen trägt.

Das Ausgangsprodukt erhält man folgendermassen:

Stufe 2.1:

30,72 g (200 mMol) β-Alanin-ethylester-hydrochlorid in 450 ml Toluol versetzt man mit 120 ml (240 mMol)

20%igem Phosgen in Toluol und rührt 6 Stunden bei 120°C. Danach kühlt man ab und setzt einen Teil des Reaktionsgemisches wie oben beschrieben weiter um.

Beispiel 3:

Analog Beispiel 1 erhält man aus 7,20 g (10 mMol) N-[2-(2-Ethoxy-ethoxy)-ethoxycarbonyl]-desferrioxamin B und Isocyanatoessigsäureethylester N-[2-(2-Ethoxy-ethoxy)-ethoxycarbonyl]-O,O′,O″-tri-(N-[ethoxycarbonylmethyl]-carbamoyl)-desferrioxamin B als öliges hygroskopisches Harz; $R_f(1)$ = 0,10, $R_f(2)$ = 0,70, $R_f(3)$ = 0,80, $R_f(4)$ = 0,90, $R_f$(HPLC) = 8,05 Minuten (Edukt: 5,70 Minuten).

Das Ausgangsprodukt erhält man folgendermassen:

Stufe 3.1:

Analog Stufe 1.1 setzt man 13,6 ml (100 mMol) Diethylenglykol-monoethylether zu einer Chlorameisensäure-[2-(2-ethoxy-ethoxy)-ethyl]-ester enthaltenden Reaktionslösung um, von der ein Teil nach dem Abkühlen in Stufe 3.2 weiterverwendet wird.

Stufe 3.2:

Analog Stufe 1.2 setzt man 26,24 g (40 mMol) Desferrioxamin B-methansulfonat zunächst mit 61 ml (480 mMol) Trimethylchlorsilan und dann mit 150 ml (50 mMol) der gemäss Stufe 3.1 erhaltenen Reaktionslösung um, rührt nicht 2, wie in Stufe 1.2, sondern 20 Stunden bei Raumtemperatur und arbeitet analog Stufe 1.2 auf. Man erhält N-[2-(2-Ethoxy-ethoxy)-ethoxycarbonyl]-desferrioxamin B; Smp. 138-139°C.

Beispiel 4:

Analog Beispiel 2 erhält man aus 3,60 g (5 mMol) N-[2-(2-Ethoxy-ethoxy)-ethoxycarbonyl]-desferrioxamin B (s. Stufe 3.2) N-[2-(2-Ethoxy-ethoxy)-ethoxycarbonyl]-O,O′,O″-tri-(N-[2-ethoxycarbonyl-ethyl]-carbamoyl)-desferrioxamin B als hygroskopisches öliges Harz; $R_f(1)$ = 0,10, $R_f(2)$ = 0,70, $R_f(3)$ = 0,80, $R_f(4)$ = 0,95, $R_f$(HPLC) = 8,32 Minuten (Edukt: 5,70 Minuten).

Beispiel 5:

Analog Beispiel 2 erhält man aus 7,48 g (10 mMol) N-[2-(2-Butoxy-ethoxy)-ethoxy-carbonyl]-desferrioxamin B N-[2-(2-Butoxy-ethoxy)-ethoxy-carbonyl]-O,O′,O″-tri-(N-[2-ethoxycarbonyl-ethyl]-carbamoyl)desferrioxamin B als hygroskopisches öliges Harz; $R_f(1)$ = 0,10, $R_f(2)$ = 0,70, $R_f(3)$ = 0,80, $R_f(4)$ = 0,95, $R_f$(HPLC) = 9,03 Minuten (Edukt: 6,74 Minuten).

Aus späteren Fraktionen der Säulenchromatographie erhält man die entsprechende Verbindung, die jedoch nur zwei, statt 3 Carbamoylgruppen trägt.

Das Ausgangsprodukt erhält man folgendermassen:

Stufe 5.1:

25,5 ml (150 mMol) Diethylenglykolmonobutylether werden analog Stufe 1.1 umgesetzt. Die Reaktionslösung enthält Chlorameisensäure-[2-(2-butoxy-ethoxy)-ethyl]-ester.

Stufe 5.2:

Analog Stufe 1.2 setzt man 45,92 g (70 mMol) Desferrioxamin B-methansulfonat in 800 ml Pyridin zunächst mit 53 ml (420 mMol) Trimethylchlorsilan und dann mit 250 ml (90 mMol) der gemäss Stufe 5.1 erhaltenen Reaktionslösung um, rührt nicht 2, wie in Stufe 1.2, sondern 6 Stunden und arbeitet analog Stufe 1.2, jedoch Kristallisation aus 800 statt 500 ml Wasser, auf. Man erhält N-[2-(2-Butoxy-ethoxy)-ethoxy-carbonyl]-desferrioxamin B; Smp. 140-141°C, $R_f$(HPLC) = 6,74 Minuten.

Beispiel 6:

Analog Beispiel 1 erhält man aus 7,48 g (10 mMol) N-[2-(2-Butoxy-ethoxy)-ethoxy-carbonyl]-desferrioxamin B (s. Stufe 5.2) N-[2-(2-Butoxy-ethoxy)-ethoxy-carbonyl]-O,O′,O″-tri-(N-[ethoxycarbonylmethyl]-carbamoyl)-desferrioxamin B in Form eines stark hygroskopischen, weissen Schaumes; $R_f(1) = 0,15$, $R_f(2) = 0,75$, $R_f(3) = 0,85$, $R_f(4) = 0,90$, $R_f$(HPLC) = 8,85 Minuten (Edukt: 6,74 Minuten).

Beispiel 7:

Analog Beispiel 2 erhält man aus 6,90 g (10 mMol) N-(Ethoxy-carbonyl-methoxycarbonyl)-desferrioxamin B N-(Ethoxycarbonyl-methoxy-carbonyl)-O,O′,O″-tri-(N-[2-ethoxycarbonyl-ethyl]-carbamoyl)-desferrioxamin B als hygroskopisches, öliges Harz; $R_f(1) = 0,05$, $R_f(2) = 0,70$, $R_f(3) = 0,80$, $R_f(4) = 0,95$, $R_f$(HPLC) = 8,29 Minuten (Edukt: 5,89 Minuten).

Aus späteren Fraktionen der Säulenchromatographie erhält man die entsprechende Verbindung, die jedoch nur zwei, statt 3 Carbamoylgruppen trägt.

Das Ausgangsprodukt erhält man folgendermassen:

Stufe 7:1:

Analog Stufe 1.1 versetzt man 14 ml (150 mMol) Glykolsäureethylester in 300 ml Toluol mit 75 ml (150 mMol) 20%igem Phosgen in Toluol, rührt 3 Stunden bei 100°C und kühlt ab. Die Reaktionslösung enthält Chlorameisensäure-(ethoxycarbonyl-methyl)-ester.

Stufe 7.2:

Analog Stufe 5.2 setzt man 45,92 g (70 mMol) Desferrioxamin B-methansulfonat zunächst mit Trimethylchlorsilan um, tropft dann 240 ml (90 mMol) der gemäss Stufe 7.1 erhaltenen Reaktionslösung innerhalb von 30 Minuten zu, rührt 5 Stunden bei Raumtemperatur, versetzt mit weiteren 50 ml (20 mMol) der gemäss Stufe 7.1 erhaltenen Lösung, rührt über Nacht, versetzt nochmals mit 50 ml (20 mMol) der Lösung, rührt 2 Stunden bei Raumtemperatur, desaktiviert mit Methanol, dampft ein, kristallisiert den Rückstand aus 300 ml Wasser, nimmt die Kristalle in 1000 ml Essigsäureethylester auf, verrührt und filtriert. Man erhält N-(Ethoxycarbonyl-methoxycarbonyl)-desferrioxamin B; Smp. 111-112°C, $R_f$(HPLC) = 5,89 Minuten.

Beispiel 8:

Analog Beispiel 1 erhält man aus 7,48 g (10 mMol) N-(Ethoxycarbonyl-methoxycarbonyl]-desferrioxamin B (s. Stufe 7.2) N-(Ethoxycarbonyl-methoxycarbonyl)-O,O′,O″-tri-(N-[ethoxycarbonylmethyl]-carbamoyl)-desferrioxamin B (isoliert aus den Fraktionen 7-12 der Säulenchromatographie) in Form eines hygroskopischen weissen Schaumes; $R_f(1) = 0,05$, $R_f(2) = 0,70$, $R_f(3) = 0,85$, $R_f(4) = 0,90$, $R_f$(HPLC) = 8,09 Minuten (Edukt: 5,89 Minuten).

Aus den Fraktionen 13-17 erhält man die entsprechende Verbindung, die jedoch nur zwei, statt 3 Carbamoylgruppen trägt.

Beispiel 9:

Analog Beispiel 2 erhält man aus N-(tert. Butyloxycarbonyl)-desferrioxamin B N-(tert.Butyloxycarbonyl-O,O′,O″-tri-(N-[2-ethoxycarbonyl-ethyl]-carbamoyl)-desferrioxamin B in Form eines amorphen, glasartig zerfallenden Schaums; $R_f$ (HPLC) = 9,05 Minuten (Edukt: 6,66 Minuten).

Das Ausgangsprodukt erhält man folgendermassen:

Stufe 9.1:

Aus N,O,O′,O″-Tetra-(trimethylsilyl)-desferrioxamin B (erhalten gemäss Stufe 1.2) und tert. Butansäureanhydrid erhält man N-(tert.-Butyloxycarbonyl)-desferrioxamin B; Smp. 136-137°C, $R_f(3) = 0,20$.

Beispiel 10:

Kapseln, enthaltend 0,25 g Wirkstoff, z.B. einer der in den Beispielen 1 - 9 beschriebenen Verbindung der Formel I, können wie folgt hergestellt werden:

Zusammensetzung (für 1000 Kapseln):

| | |
|---|---|
| Wirkstoff | 250 g |
| Talk | 36 g |
| Weizenstärke | 24 g |
| Magnesiumstearat | 16 g |
| Laktose | 4 g |
| | 330 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt, welche mit einem Magensaft-resistenten Ueberzug versehen sind.

## Patentansprüche

## Patentansprüche für folgende Vertragsstaaten : AT, BE, CH, DE, FR, GB, IT, LI, LU, NL, SE

1. Eine Verbindung der Formel I,

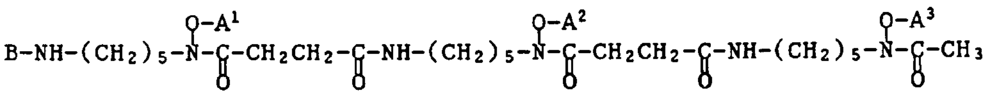

(I)

in welcher mindestens einer der Reste $A^1$, $A^2$ und $A^3$ für einen Carbamoylrest der Teilformel -CO-NH-Alk-CO-O-$R_a^1$ (II) steht, worin $R_a^1$ $C_1$-$C_4$-Alkyl oder $C_2$-$C_4$-Alkenyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, und die übrigen der Symbole $A^1$, $A^2$ und $A^3$ unabhängig voneinander für Wasserstoff oder einen von einer Carbonsäure abgeleiteten Acylrest Ac stehen, und B für Wasserstoff oder einen Acylrest Ac steht, welcher von Alkyl-O-($CH_2$-$CH_2$-O-)$_q$CO-, worin q eine Zahl grösser als 5 bedeutet, verschieden ist, oder ein Salz einer solchen Verbindung mit salzbildenden Eigenschaften.

2. Eine Verbindung gemäss Anspruch 1 der Formel I, worin $A^1$, $A^2$ und $A^3$ je ein und denselben Carbamoylrest der Teilformel II und B einen Acylrest bedeuten, welcher von Alkyl-O-($CH_2$-$CH_2$-O-)$_q$CO-, worin q eine Zahl grösser als 5 bedeutet, verschieden ist.

3. Eine Verbindung gemäss Anspruch 1 oder 2 der Formel I, in welcher B $C_2$-$C_{10}$-Alkanoyl, ($C_1$-$C_9$-Alkoxy)-carbonyl, Diniederalkylamino-carbonyl, lineares Mono-, Di- oder Tri-oxa-alkoxy-carbonyl mit bis zu 18 Kettengliedern oder ω-($C_1$-$C_4$-Alkoxycarbonyl)-niederalkoxycarbonyl bedeutet.

4. Eine Verbindung gemäss einem der Ansprüche 1-3 der Formel I, worin im Rest der Teilformel II $R_a^1$ einen linearen $C_1$-$C_4$-Alkylrest und Alk einen linearen $C_1$-$C_7$-Alkylenrest, dessen freie Valenzen von beiden endständigen C-Atomen ausgehen, darstellen.

5. Eine Verbindung nach Anspruch 1 der Formel I, in welcher $A^1$, $A^2$ und $A^3$ die gleiche Bedeutung haben und jeweils für einen Carbamoylrest der Teilformel II stehen, worin $R_a^1$ $C_1$-$C_4$-Alkyl und Alk $C_1$-$C_5$-Alkylen bedeuten, und B für 2-($C_2$-$C_4$-Alkoxy)-ethoxycarbonyl, 2-[2-($C_2$-$C_4$-Alkoxy)-ethoxy]-ethoxycarbonyl oder Ethoxycarbonylmethoxycarbonyl steht.

6. Eine Verbindung nach Anspruch 1 der Formel I, in welcher $A^1$, $A^2$ und $A^3$ die gleiche Bedeutung haben und jeweils für einen Carbamoylrest der Teilformel II stehen, worin $R_a^1$ $C_1$-$C_4$-Alkyl und Alk Methylen oder Ethylen bedeuten, und B für 2-($C_2$-$C_4$-Alkoxy)-ethoxycarbonyl, 2-[2-($C_2$-$C_4$-Alkoxy)-ethoxy]-ethoxycarbonyl, Ethoxycarbonylmethoxycarbonyl oder ($C_1$-$C_4$-Alkoxy)-carbonyl steht.

7. Eine Verbindung gemäss einem der Ansprüche 1-3 und 5 der Formel I, worin im Rest der Teilformel II $R_a^1$ ein lineares $C_1$-$C_4$-Alkyl und Alk ein lineares oder einmal verzweigtes $C_1$-$C_5$-1,1-Alkylen darstellen.

8. Eine Verbindung gemäss einem der Ansprüche 1-5 der Formel I, worin im Rest der Teilformel II $R_a^1$ ein lineares $C_1$-$C_4$-Alkyl und Alk Methylen oder Ethylen darstellen.

9. Eine Verbindung nach Anspruch 1 der Formel I, ausgewählt aus der Gruppe, bestehend aus
N-(2-Butoxy-ethoxy-carbonyl)-O,O′,O″-tri-(N-[ethoxycarbonylmethyl]-carbamoyl)-desferrioxamin B,
N-(2-Butoxy-ethoxy-carbonyl)-O,O′,O″-tri-(N-[2-ethoxycarbonyl-ethyl]-carbamoyl)-desferrioxamin B,
N-[2-(2-Ethoxy-ethoxy)ethoxycarbonyl]-O,O′,O″-tri-(N-[ethoxycarbonylmethyl]-carbamoyl)-desferrioxamin B,
N-[2-(2-Ethoxy-ethoxy)-ethoxycarbonyl]-O,O′,O″-tri-(N-[2-ethoxycarbonyl-ethyl]-carbamoyl)-desferrioxamin B,
N-[2-(2-Butoxy-ethoxy)-ethoxy-carbonyl]-O,O′,O″-tri-(N-[2-ethoxycarbonyl-ethyl]-carbamoyl)-desferrioxamin B,
N-[2-(2-Butoxy-ethoxy)-ethoxy-carbonyl]-O,O′,O″-tri-(N-[ethoxycarbonylmethyl]-carbamoyl)-desferrioxamin B,
N-(Ethoxycarbonyl-methoxycarbonyl)-O,O′,O″-tri-(N-[2-ethoxycarbonyl-ethyl]-carbamoyl)-desferrioxamin B,
N-(Ethoxycarbonyl-methoxycarbonyl)-O,O′,O″-tri-(N-[ethoxycarbonylmethyl]-carbamoyl)-desferrioxamin B und
N-(tert.Butyloxycarbonyl-O,O′,O″-tri-(N-[2-ethoxycarbonyl-ethyl]-carbamoyl)-desferrioxamin B.

10. Eine Verbindung gemäss einem der Ansprüche 1-9 zur Anwendung in einem Verfahren zur therapeutischen Behandlung des menschlichen oder tierischen Körpers.

11. Eine pharmazeutische Zusammensetzung, enthaltend neben pharmazeutischem Trägermaterial als Wirkstoff eine Verbindung der Formel I nach einem der Ansprüche 1-9 oder ein pharmazeutisch verwendbares Salze einer solchen Verbindung mit salzbildenden Eigenschaften.

12. Verwendung einer Verbindung gemäss einem der Ansprüche 1-9 zur Herstellung von pharmazeutischen Zusammensetzungen, die für die Anwendung zur Behandlung von krankhaften Zuständen in Warmblütern einschliesslich des Menschen, welche mit einem Ueberschuss an Eisen(III) oder Aluminium im Körper zusammenhängen oder durch Eisen(III)-abhängige pathogene Organismen verursacht werden, bestimmt sind.

13. Verfahren zur Herstellung einer Verbindung der Formel I nach Anspruch 1 oder eines Salzes einer solchen Verbindung mit salzbildenden Eigenschaften, dadurch gekennzeichnet, dass man

a) ein Derivat von Desferrioxamin B der Formel IV,

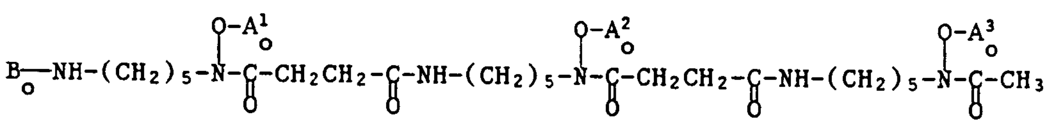

(IV)

worin $B_o$ ein Acylrest Ac, welcher von Alkyl-O-($CH_2$-$CH_2$-O)$_q$CO-, worin q eine Zahl grösser als 5 bedeutet, verschieden ist, oder eine Aminoschutzgruppe ist und mindestens eines der Symbole $A_o^1$, $A_o^2$ und $A_o^3$ Wasserstoff bedeutet und die übrigen der Symbole $A_o^1$, $A_o^2$ und $A_o^3$ einen oben definierten Acylrest Ac bedeuten, mit einem Isocyanatocarbonsäureester der Formel III,

$$O{=}C{=}N\text{-}Alk\text{-}CO\text{-}O\text{-}R_a^1 \qquad (III)$$

worin $R_a^1$ und Alk die oben genannten Bedeutungen haben, wobei, falls im Rest Alk Amino- oder Hydroxygruppen vorhanden sind, diese durch leicht abspaltbare Schutzgruppen geschützt sind, umsetzt, und vorhandene Schutzgruppen abspaltet, oder

b) zur Herstellung einer Verbindung der Formel I, worin B für einen Acylrest steht, welcher von Alkyl-O-($CH_2$-$CH_2$-O-)$_q$CO-, worin q eine Zahl grösser als 5 bedeutet, verschieden ist, eine Verbindung der Formel I, worin B für Wasserstoff oder eine Silylgruppe steht, acyliert, und, wenn erwünscht, nach Durchführung der Verfahren a) oder b) eine erhaltene freie Verbindung der Formel I mit salzbildenden Eigenschaften in eines ihrer Salze umwandelt oder eine Verbindung der Formel I aus einem ihrer Salze freisetzt.

Patentansprüche für folgende Vertragsstaaten : ES, GR

1. Verfahren zur Herstellung einer Verbindung der Formel I,

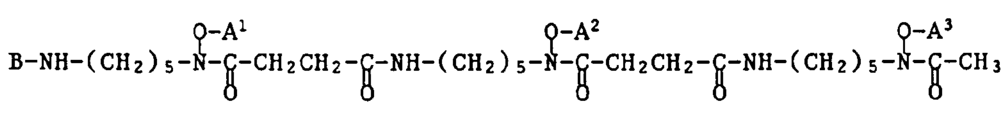

(I)

in welcher mindestens einer der Reste $A^1$, $A^2$ und $A^3$ für einen Carbamoylrest der Teilformel -CO-NH-Alk-CO-O-$R_a^1$ (II) steht, worin $R_a^1$ $C_1$-$C_4$-Alkyl oder $C_2$-$C_4$-Alkenyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, und die übrigen der Symbole $A^1$, $A^2$ und $A^3$ unabhängig voneinander für Wasserstoff oder einen von einer Carbonsäure abgeleiteten Acylrest Ac stehen, und B für Wasserstoff oder einen Acylrest Ac steht, welcher von Alkyl-O-($CH_2$-$CH_2$-O-)$_q$CO-, worin q eine Zahl grösser als 5 bedeutet, verschieden ist, oder eines Salzes einer solchen Verbindung mit salzbildenden Eigenschaften, dadurch gekennzeichnet, dass man

a) ein Derivat von Desferrioxamin B der Formel IV,

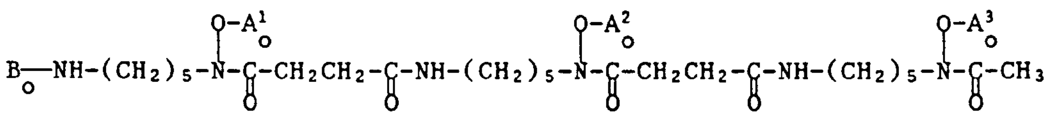

(IV)

worin $B_o$ ein Acylrest Ac, welcher von Alkyl-O-($CH_2$-$CH_2$-O)$_q$CO-, worin q eine Zahl grösser als 5 bedeutet, verschieden ist, oder eine Aminoschutzgruppe ist und mindestens eines der Symbole $A_o^1$, $A_o^2$ und $A_o^3$ Wasserstoff bedeutet und die übrigen der Symbole $A_o^1$, $A_o^2$ und $A_o^3$ einen oben definierten Acylrest Ac bedeuten, mit einem Isocyanatocarbonsäureester der Formel III,

$$O{=}C{=}N\text{-}Alk\text{-}CO\text{-}O\text{-}R_a^1 \qquad \text{(III)}$$

worin $R_a^1$ und Alk die oben genannten Bedeutungen haben, wobei, falls im Rest Alk Amino- oder Hydroxygruppen vorhanden sind, diese durch leicht abspaltbare Schutzgruppen geschützt sind, umsetzt, und vorhandene Schutzgruppen abspaltet, oder

b) zur Herstellung einer Verbindung der Formel I, worin B für einen Acylrest steht, welcher von Alkyl-O-($CH_2$-$CH_2$-O-)$_q$CO-, worin q eine Zahl grösser als 5 bedeutet, verschieden ist, eine Verbindung der Formel I, worin B für Wasserstoff oder eine Silylgruppe steht und $A^1$, $A^2$ und $A^3$ die obengenannten Bedeutungen haben, acyliert, und, wenn erwünscht, nach Durchführung der Verfahren a) oder b) eine erhaltene freie Verbindung der Formel I mit salzbildenden Eigenschaften in eines ihrer Salze umwandelt oder eine Verbindung der Formel I aus einem ihrer Salze freisetzt.

2. Verfahren gemäss Anspruch 1, dadurch gekennzeichnet, dass man die Ausgangsstoffe so wählt, dass man eine Verbindung der Formel I, worin $A^1$, $A^2$ und $A^3$ je ein und denselben Carbamoylrest der Teilformel II und B einen Acylrest bedeuten, welcher von Alkyl-O-($CH_2$-$CH_2$-O-)$_q$CO-, worin q eine Zahl grösser als 5 bedeutet, verschieden ist, herstellt.

3. Verfahren gemäss Anspruch 1 oder 2, dadurch gekennzeichnet, dass man die Ausgangsstoffe so wählt, dass man eine Verbindung der Formel I, in welcher B $C_2$-$C_{10}$-Alkanoyl, ($C_1$-$C_9$-Alkoxy)-carbonyl, Diniederalkylamino-carbonyl, lineares Mono-, Di- oder Tri-oxa-alkoxy-carbonyl mit bis zu 18 Kettengliedern oder ω-($C_1$-$C_4$-Alkoxycarbonyl)-niederalkoxycarbonyl bedeutet, herstellt.

4. Verfahren gemäss einem der Ansprüche 1-3, dadurch gekennzeichnet, dass man die Ausgangsstoffe so wählt, dass man eine Verbindung der Formel I, worin im Rest der Teilformel II $R_a^1$ einen linearen $C_1$-$C_4$-Alkylrest und Alk einen linearen $C_1$-$C_7$-Alkylenrest, dessen freie Valenzen von beiden endständigen C-Atomen ausgehen, darstellen, herstellt.

5. Verfahren nach Anspruch 1, dadurch gekennzeichnet, dass man die Ausgangsstoffe so wählt, dass man eine Verbindung der Formel I, in welcher $A^1$, $A^2$ und $A^3$ die gleiche Bedeutung haben und jeweils für einen Carbamoylrest der Teilformel II stehen, worin $R_a^1$ $C_1$-$C_4$-Alkyl und Alk $C_1$-$C_5$-Alkylen bedeuten, und B für 2-($C_2$-$C_4$-Alkoxy)-ethoxycarbonyl, 2-[2-($C_2$-$C_4$-Alkoxy)-ethoxy]-ethoxycarbonyl oder Ethoxycarbonylmethoxycarbonyl steht, herstellt.

6. Verfahren nach Anspruch 1, dadurch gekennzeichnet, dass man die Ausgangsstoffe so wählt, dass man eine Verbindung der Formel I, in welcher $A^1$, $A^2$ und $A^3$ die gleiche Bedeutung haben und jeweils für einen Carbamoylrest der Teilformel II stehen, worin $R_a^1$ $C_1$-$C_4$-Alkyl und Alk Methylen oder Ethylen bedeuten, und B für 2-($C_2$-$C_4$-Alkoxy)-ethoxycarbonyl, 2-[2-($C_2$-$C_4$-Alkoxy)-ethoxy]-ethoxycarbonyl, Ethoxycarbonylmethoxycarbonyl oder ($C_1$-$C_4$-Alkoxy)-carbonyl steht, herstellt.

7. Verfahren gemäss einem der Ansprüche 1-3 und 5, dadurch gekennzeichnet, dass man die Ausgangsstoffe so wählt, dass man eine Verbindung der Formel I, worin im Rest der Teilformel II $R_a^1$ ein lineares $C_1$-$C_4$-

Alkyl und Alk ein lineares oder einmal verzweigtes $C_1$-$C_5$-1,1-Alkylen darstellen, herstellt.

8. Verfahren gemäss einem der Ansprüche 1-5, dadurch gekennzeichnet, dass man die Ausgangsstoffe so wählt, dass man eine Verbindung der Formel I, worin im Rest der Teilformel II $R_a^1$ ein lineares $C_1$-$C_4$-Alkyl und Alk Methylen oder Ethylen darstellen, herstellt.

9. Verfahren nach Anspruch 1, dadurch gekennzeichnet, dass man die Ausgangsstoffe so wählt, dass man N-(2-Butoxy-ethoxy-carbonyl)-O,O′,O″-tri-(N-[ethoxycarbonylmethyl]-carbamoyl)-desferrioxamin B herstellt.

10. Verfahren nach Anspruch 1, dadurch gekennzeichnet, dass man die Ausgangsstoffe so wählt, dass man N-(2-Butoxy-ethoxy-carbonyl)-O,O′,O″-tri-(N-[2-ethoxycarbonyl-ethyl]-carbamoyl)-desferrioxamin B herstellt.

11. Verfahren nach Anspruch 1, dadurch gekennzeichnet, dass man die Ausgangsstoffe so wählt, dass man N-[2-(2-Ethoxy-ethoxy)-ethoxycarbonyl]-O,O′,O″-tri-(N-[ethoxycarbonylmethyl]-carbamoyl)-desferrioxamin B herstellt.

12. Verfahren nach Anspruch 1, dadurch gekennzeichnet, dass man die Ausgangsstoffe so wählt, dass man N-[2-(2-Ethoxy-ethoxy)-ethoxycarbonyl]-O,O′,O″-tri-(N-[2-ethoxycarbonyl-ethyl]-carbamoyl)-desferrioxamin B herstellt.

13. Verfahren nach Anspruch 1, dadurch gekennzeichnet, dass man die Ausgangsstoffe so wählt, dass man eine Verbindung, ausgewählt aus der Gruppe, bestehend aus
N-[2-(2-Butoxy-ethoxy)-ethoxy-carbonyl]-O,O′,O″-tri-(N-[2-ethoxycarbonyl-ethyl]-carbamoyl)-desferrioxamin B,
N-[2-(2-Butoxy-ethoxy)-ethoxy-carbonyl]-O,O′,O″-tri-(N-[ethoxycarbonylmethyl]-carbamoyl)-desferrioxamin B,
N-(Ethoxycarbonyl-methoxycarbonyl)-O,O′,O″-tri-(N-[2-ethoxycarbonylethyl]-carbamoyl)-desferrioxamin B,
N-(Ethoxycarbonyl-methoxycarbonyl)-O,O′,O″-tri-(N-[ethoxycarbonylmethyl]-carbamoyl)-desferrioxamin B und
N-(tert.Butyloxycarbonyl-O,O′,O″-tri-(N-[2-ethoxycarbonyl-ethyl]-carbamoyl)-desferrioxamin B herstellt.

## Claims

### Claims for the following Contracting States : AT, BE, CH, DE, FR, GB, IT, LI, LU, NL, SE

1. A compound of formula I

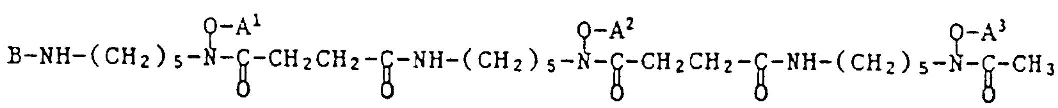

(I)

in which at least one of the radicals $A^1$, $A^2$ and $A^3$ is a carbamoyl radical of the partial formula -CO-NH-Alk-CO-O-$R_a^1$ (II) in which $R_a^1$ is $C_1$-$C_4$alkyl or $C_2$-$C_4$alkenyl and Alk is $C_1$-$C_7$alkylene that is unsubstituted or substituted by hydroxy, $C_1$-$C_4$alkanoyloxy, amino, $C_1$-$C_4$alkoxycarbonyl, carbamoyl, phenyl, hydroxyphenyl, methoxyphenyl or by indolyl, and the remainder of the symbols $A^1$, $A^2$ and $A^3$ are each, independently of one another, hydrogen or an acyl radical Ac derived from a carboxylic acid, and B is hydrogen or an acyl radical Ac other than alkyl-O-($CH_2$-$CH_2$-O-)$_q$CO- in which q is an integer greater than 5, or a salt of such a compound having salt-forming properties.

2. A compound according to claim 1 of formula I, in which $A^1$, $A^2$ and $A^3$ are each one and the same carbamoyl radical of the partial formula II and B is an acyl radical that is other than alkyl-O-($CH_2$-$CH_2$-O-)$_q$CO- in which q is an integer greater than 5.

3. A compound according to claim 1 or 2 of formula I, in which B is $C_2$-$C_{10}$alkanoyl, ($C_1$-$C_6$alkoxy)-carbonyl, di- lower alkylaminocarbonyl, linear mono-, di- or tri-oxaalkoxycarbonyl having up to 18 chain members or ω-($C_1$-$C_4$alkoxycarbonyl)-lower alkoxycarbonyl.

4. A compound according to any one of claims 1 to 3 of formula I, in which in the radical of the partial formula II $R_a^1$ is a linear $C_1$-$C_4$alkyl radical and Alk is a linear $C_1$-$C_7$alkylene radical, the free valencies of which originate from the two terminal carbon atoms.

5. A compound according to claim 1 of formula I, in which $A^1$, $A^2$ and $A^3$ have the same meaning and are each a carbamoyl radical of the partial formula II in which $R_a^1$ is $C_1$-$C_4$alkyl and Alk is $C_1$-$C_5$alkylene, and B is 2-($C_2$-$C_4$alkoxy)-ethoxycarbonyl, 2-[2-($C_2$-$C_4$alkoxy)-ethoxy]ethoxycarbonyl or ethoxycarbonylmethoxycarbonyl.

6. A compound according to claim 1 of formula I, in which $A^1$, $A^2$ and $A^3$ have the same meaning and are

each a carbamoyl radical of the partial formula II in which $R_a^1$ is $C_1$-$C_4$alkyl and Alk is methylene or ethylene, and B is 2-($C_2$-$C_4$alkoxy)-ethoxycarbonyl, 2-[2-($C_2$-$C_4$alkoxy)ethoxy]-ethoxycarbonyl, ethoxycarbonylmethoxycarbonyl or ($C_1$-$C_4$alkoxy)-carbonyl.

7. A compound according to any one of claims 1 to 3 and 5 of formula I, in which in the radical of the partial formula II $R_a^1$ is a linear $C_1$-$C_4$alkyl radical and Alk is a $C_1$-$C_5$-1,1-alkylene radical that is linear or branched once.

8. A compound according to any one of claims 1 to 5 of formula I, in which in the radical of the partial formula II $R_a^1$ is a linear $C_1$-$C_4$alkyl radical and Alk is methylene or ethylene.

9. A compound according to claim 1 of the formula I selected from the group consisting of
N-(2-butoxyethoxycarbonyl)-O,O′,O″-tri-(N-[ethoxycarbonylmethyl]-carbamoyl)-desferrioxamine B,
N-(2-butoxyethoxycarbonyl)-O,O′,O″-tri-(N-[2-ethoxycarbonylethyl]-carbamoyl)-desferrioxamine B,
N-[2-(2-ethoxyethoxy)-ethoxycarbonyl]-O,O′,O″-tri-(N-[ethoxycarbonylmethyl]-carbamoyl)-desferrioxamine B,
N-[2-(2-ethoxyethoxy)-ethoxycarbonyl]-O,O′,O″-tri-(N-[2-ethoxycarbonylethyl]-carbamoyl)-desferrioxamine B,
N-[2-(2-butoxyethoxy)-ethoxycarbonyl]-O,O′,O″-tri-(N-[2-ethoxycarbonylethyl]-carbamoyl)-desferrioxamine B,
N-[2-(2-butoxyethoxy)-ethoxycarbonyl]-O,O′,O″-tri-(N-[ethoxycarbonylmethyl]-carbamoyl)-desferrioxamine B,
N-(ethoxycarbonylmethoxycarbonyl)-O,O′,O″-tri-(N-[2-ethoxycarbonylethyl]-carbamoyl)-desferrioxamine B,
N-(ethoxycarbonylmethoxycarbonyl)-O,O′,O″-tri-(N-[ethoxycarbonylmethyl]-carbamoyl)-desferrioxamine B and
N-(tert-butoxycarbonyl-O,O′,O″-tri-(N-[2-ethoxycarbonylethyl]-carbamoyl)-desferrioxamine B.

10. A compound according to any one of claims 1 to 9, for use in a method for the therapeutic treatment of the human or animal body.

11. A pharmaceutical composition comprising as active ingredient a compound of formula I according to any one of claims 1 to 9, or a pharmaceutically acceptable salt of such a compound having salt-forming properties, together with a pharmaceutical carrier.

12. The use of a compound according to any one of claims 1 to 9 for the preparation of a pharmaceutical composition that is intended for use in the treatment of pathological conditions in warm-blooded animals, including humans, that are associated with an excess of iron(III) or aluminium in the body or are caused by iron(III)-dependent pathogenic organisms.

13. A process for the preparation of a compound of formula I according to claim 1, or a salt of such a compound having salt-forming properties, which comprises

a) reacting a derivative of desferrioxamine B of formula IV

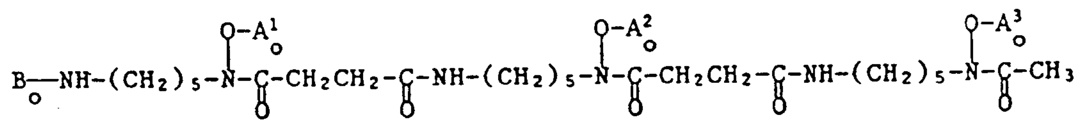

(IV)

in which $B_o$ is an acyl radical Ac that is other than alkyl-O-($CH_2$-$CH_2$-O-)$_q$CO- in which q is an integer greater than 5, or in which $B_o$ is an amino-protecting group, and at least one of the symbols $A_o^1$, $A_o^2$ and $A_o^3$ is hydrogen, the remainder of the symbols $A_o^1$, $A_o^2$ and $A_o^3$ being an above-defined acyl radical Ac, with an isocyanatocarboxylic acid ester of formula III

$$O=C=N\text{-Alk-CO-O-}R_a^1 \qquad \text{(III)}$$

in which $R_a^1$ and Alk have the meanings given above, wherein if any amino or hydroxy groups are present in the radical Alk they are protected by readily removable protecting groups, and removing any protecting groups present, or

b) for the preparation of a compound of formula I in which B is an acyl radical that is other than alkyl-O-($CH_2$-$CH_2$-O-)$_q$CO- in which q is an integer greater than 5, acylating a compound of formula I in which B is hydrogen or a silyl-,group and, if desired, after carrying out process a) or b), converting a resulting free compound of formula I having salt-forming properties into one of its salts or freeing a compound of formula I from one of its salts.

**Claims for the following Contracting States : ES, GR**

1. A process for the preparation of a compound of formula I

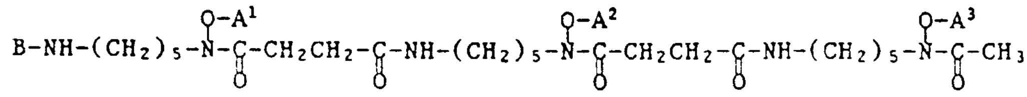

(I)

in which at least one of the radicals $A^1$, $A^2$ and $A^3$ is a carbomoyl radical of the partial formula -CO-NH-Alk-CO-O-$R_a^1$ (II) in which $R_a^1$ is $C_1$-$C_4$alkyl or $C_2$-$C_4$alkenyl and Alk is $C_1$-$C_7$alkylene that is unsubstituted or substituted by hydroxy, $C_1$-$C_4$alkanoyloxy, amino, $C_1$-$C_4$alkoxycarbonyl, carbamoyl, phenyl, hydroxyphenyl, methoxyphenyl or by indolyl, and the remainder of the symbols $A^1$, $A^2$ and $A^3$ are each, independently of one another, hydrogen or an acyl radical Ac derived from a carboxylic acid, and B is hydrogen or an acyl radical Ac other than alkyl-O-($CH_2$-$CH_2$-O-)$_q$CO- in which q is an integer greater than 5, or a salt of such a compound having salt-forming properties, which comprises

a) reacting a derivative of desferrioxamine B of formula IV

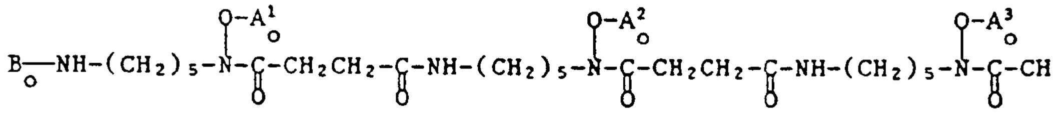

(IV)

in which $B_o$ is an acyl radical Ac that is other than alkyl-O-($CH_2CH_2$-O-)$_q$CO- in which q is an integer greater than 5, or in which $B_o$ is an amino-protecting group, and at least one of the symbols $A_o^1$, $A_o^2$ and $A_o^3$ is hydrogen, the remainder of the symbols $A_o^1$, $A_o^2$ and $A_o^3$ being an above-defined acyl radical Ac, with an isocyanatocarboxylic acid ester of formula III

$$O{=}C{=}N\text{-}Alk\text{-}CO\text{-}OR_a^1 \qquad (III)$$

in which $R_a^1$ and Alk have the meanings given above, wherein if any amino or hydroxy groups are present in the radical Alk they are protected by readily removable protecting groups, and removing any protecting groups present, or

b) for the preparation of a compound of formula I in which B is an acyl radical that is other than alkyl-O-($CH_2$-$CH_2$-O-)$_q$CO- in which q is an integer greater than 5, acylating a compound of formula I in which B is hydrogen or a silyl group and $A^1$, $A^2$ and $A^3$ have the meanings given above and, if desired, after carrying out process a) or b), converting a resulting free compound of formula I having salt-forming properties into one of its salts or freeing a compound of formula I from one of its salts.

2. A process according to claim 1 which comprises so selecting the starting materials that a compound of formula I is prepared in which $A^1$, $A^2$ and $A^3$ are each one and the same carbamoyl radical of the partial formula II and B is an acyl radical that is other than alkyl-O-($CH_2$-$CH_2$-O-)$_q$CO- in which q is an integer greater than 5.

3. A process according to claim 1 or 2, which comprises so selecting the starting materials that a compound of formula I is prepared in which B is $C_2$-$C_{10}$alkanoyl, ($C_1$-$C_9$alkoxy)-carbonyl, di-lower alkylaminocarbonyl, linear mono-, di- or tri-oxa-alkoxycarbonyl having up to 18 chain members or ω-($C_1$-$C_4$alkoxycarbonyl)-lower alkoxycarbonyl.

4. A process according to any one of claims 1 to 3, which comprises so selecting the starting materials that a compound of formula I is prepared in which in the radical of the partial formula II $R_a^1$ is a linear $C_1$-$C_4$alkyl radical and Alk is a linear $C_1$-$C_7$alkylene radical, the free valencies of which originate from the two terminal carbon atoms.

5. A process according to claim 1, which comprises so selecting the starting materials that a compound of formula I is prepared in which $A^1$, $A^2$ and $A^3$ have the same meaning and are each a carbamoyl radical of the partial formula II in which $R_a^1$ is $C_1$-$C_4$alkyl and Alk is $C_1$-$C_5$alkylene, and B is 2-($C_2$-$C_4$alkoxy)-ethoxycarbonyl, 2-[2-($C_2$-$C_4$alkoxy)-ethoxy]-ethoxycarbonyl or ethoxycarbonylmethoxycarbonyl.

6. A process according to claim 1, which comprises so selecting the starting materials that a compound of formula I is prepared in which $A^1$, $A^2$ and $A^3$ have the same meaning and are each a carbamoyl radical of the partial formula II in which $R_a^1$ is $C_1$-$C_4$alkyl and Alk is methylene or ethylene, and B is 2-($C_2$-$C_4$alkoxy)-ethoxycarbonyl, 2-[2-($C_2$-$C_4$alkoxy)-ethoxy]-ethoxycarbonyl, ethoxycarbonylmethoxycarbonyl or ($C_1$-$C_4$alkoxy)-carbonyl.

7. A process according to any one of claims 1 to 3 and 5, which comprises so selecting the starting materials

that a compound of formula I is prepared in which in the radical of the partial formula II $R_a^1$ is a linear $C_1$-$C_4$alkyl radical and Alk is a $C_1$-$C_5$-1,1-alkylene radical that is linear or branched once.

8. A process according to any one of claims 1 to 5 which comprises so selecting the starting materials that a compound of formula I is prepared in which in the radical of the partial formula II $R_a^1$ is a linear $C_1$-$C_4$alkyl radical and Alk is methylene or ethylene.

9. A process according to claim 1, which comprises so selecting the starting materials that N-(2-butoxyethoxycarbonyl)-O,O′,O″-tri-(N-[ethoxycarbonylmethyl]-carbamoyl)-desferrioxamine B is prepared.

10. A process according to claim 1, which comprises so selecting the starting materials that N-(2-butoxyethoxycarbonyl)-O,O′,O″-tri-(N-[2-ethoxycarbonylethyl]-carbamoyl)-desferrioxamine B is prepared.

11. A process according to claim 1, which comprises so selecting the starting materials that N-[2-(2-ethoxyethoxy)-ethoxycarbonyl]-O,O′,O″-tri-(N-[ethoxycarbonylmethyl]-carbamoyl)-desferrioxamine B is prepared.

12. A process according to claim 1, which comprises so selecting the starting materials that N-[2-(2-ethoxyethoxy)ethoxycarbonyl]-O,O′,O″-tri-(N-[2-ethoxycarbonylethyl]-carbamoyl)-desferrioxamine B is prepared.

13. A process according to claim 1, which comprises so selecting the starting materials that a compound selected from the group consisting of
N-[2-(2-butoxyethoxy)ethoxycarbonyl]-O,O′,O″-tri-(N-[2-ethoxycarbonylethyl]-carbamoyl-)-desferrioxamine B,
N-[2-(2-butoxyethoxy)ethoxycarbonyl]-O,O′,O″-tri-(N-[ethoxycarbonylmethyl]-carbamoyl)-desferrioxamine B,
N-(ethoxycarbonylmethoxycarbonyl)-O,O′,O″-tri-(N-[2-ethoxycarbonylethyl]-carbamoyl)-desferrioxamine B,
N-(ethoxycarbonylmethoxycarbonyl)-O,O′,O″-tri-(N-[ethoxycarbonylmethyl]-carbamoyl)-desferrioxamine B and
N-(tert-butoxycarbonyl-O,O′,O″-tri-(N-[2-ethoxycarbonylethyl]-carbamoyl)-desferrioxamine B is prepared.

## Revendications

### Revendications pour les Etats contractants suivants : AT, BE, CH, DE, FR, GB, IT, LI, LU, NL, SE

1. Composé de formule I

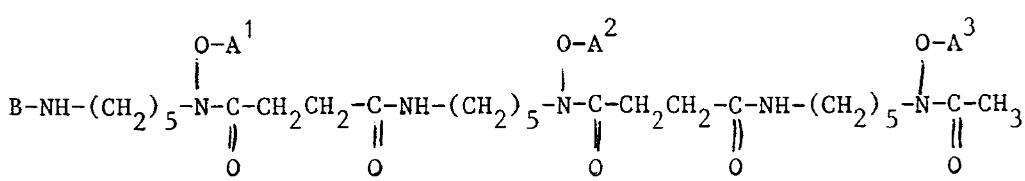

(I)

dans laquelle au moins l'un des restes $A^1$, $A^2$ et $A^3$ représente un reste carbamoyle de formule partielle -CO-NH-Alk-CO-O-$R^1_a$ (II) où $R_a^1$ représente un alkyle en $C_1$-$C_4$ ou un alcényle en $C_2$-$C_4$ et Alk représente un alkylène en $C_1$-$C_7$ non substitué ou substitué par l'hydroxyle, un alcanoyloxy en $C_1$-$C_4$, un amino, un alcoxy en $C_1$-$C_4$ carbonyle, uncarbamoyle , un phényle, un hydroxyphényle, le méthoxyphényle ou l'indolyle et les symboles $A^1$, $A^2$ et $A^3$ restants représentent indépendamment l'un de l'autre l'hydrogène ou un reste acyle Ac dérivé d'un acide carboxylique et B représente l'hydrogène ou un reste acyle Ac différent d'unalkyl -O-($CH_2$-$CH_2$-O-)$_q$CO- où q représente un nombre plus grand que 5 ou un sel d'un tel composé présentant des propriétés salifiables.

2. Composé de formule I selon la revendication 1 où $A^1$, $A^2$ et $A^3$ représentent chacun un seul et même reste carbamoyle de formule partielle II et B représente un reste acyle, différent de alkyl -O-($CH_2$-$CH_2$-O-)$_q$CO-, q représentant un nombre plus grand que 5.

3. Composé de formule I selon la revendication 1 ou 2 dans laquelle B représente un alcanoyle en $C_2$-$C_{10}$, un (alcoxy en $C_1$-$C_9$)carbonyle, un dialkyle inférieur amino-carbonyle, un mono-, di- ou tri-oxa-alcoxy-carbonyle linéaire ayant jusqu'à 18 chaînons ou un ω-(alcoxy en $C_1$-$C_4$ carbonyl)-alcoxy inférieur carbonyle.

4. Composé de formule I selon l'une des revendications 1 à 3 où dans le reste de formule partielle II $R_a^1$ représente un reste alkyle en $C_1$-$C_4$ linéaire et Alk représente un reste alkylène en $C_1$-$C_7$ linéaire dont les valences libres partent des deux atomes de C terminaux.

5. Composé de formule I selon la revendication 1 dans laquelle $A^1$, $A^2$ et $A^3$ ont la même signification et

représentent un reste carbamoyle de formule partielle II où $R_a^1$ représente un alkyle en $C_1$-$C_4$ et Alk un alkylène en $C_1$-$C_5$ et B un 2-(alcoxy en $C_2$-$C_4$)éthoxycarbonyle, un 2-[2-(alcoxy en $C_2$-$C_4$)-éthoxy]-éthoxycarbonyle ou l'éthoxycarbonylméthoxycarbonyle.

6. Composé de formule I selon la revendication 1 dans laquelles $A^1$, $A^2$ et $A^3$ ont la même signification et représentent un reste carbamoyle de formule partielle II où $R_a^1$ représente un alkyle en $C_1$-$C_4$ et Alk le méthylène ou l'éthylène et B un 2-(alcoxy en $C_2$-$C_4$)-éthoxycarbonyle, un 2-[2-(alcoxy en $C_2$-$C_4$)-éthoxy]éthoxycarbonyle, l'éthoxycarbonylméthoxycarbonyle ou un (alcoxy en $C_1$-$C_4$)-carbonyle.

7. Composé de formule I selon l'une des revendications 1 à 3 et 5 où dans le reste de formule partielle II $R_a^1$ représente un alkyle en $C_1$-$C_4$ linéaire et Alk un 1,1-alkylène en $C_1$-$C_5$ linéaire ou ramifié une fois.

8. Composé de formule I selon l'une des revendications 1 à 5 où dans le reste de formule partielle II $R_a^1$ représente un alkyle en $C_1$-$C_4$ linéaire et Alkleéthylène ou l'éthylène.

9. Composé de formule I selon la revendication 1 choisi dans le groupe constitué par
la N-(2-butoxy-éthoxy-carbonyl)-O,O′,O″-tri-(N-[éthoxy-carbonylméthyl]-carbomoyl)-desferrioxamine B,
la N-(2-butoxy-éthoxy-carbonyl)-O,O′,O″-tri-(N-[2-éthoxycarbonyléthyl]-carbamoyl)-desferrioxamine B,
la N-[2-(2-éthoxy-éthoxy)-éthoxycarbonyl]-O,O′,O″-tri-(N-[éthoxycarbonyl-méthyl]-carbamoyl)-desferrioxamine B,
la N-[2-(2-éthoxy-éthoxy)-éthoxycarbonyl]-O,O′,O″-tri-(N-[2-éthoxycarbonyl-éthyl]-carbamoyl)-desferrioxamine B,
la N-[2-(2-butoxy-éthoxy)-éthoxycarbonyl]-O,O′,O″-tri-(N-[2-éthoxycarbonyl(2-éthyl]-carbamoyl)-desferrioxamine B,
la N-[2-(2-butoxy-éthoxy)-éthoxycarbonyl]-O,O′,O″-tri-(N-[éthoxycarbonyl-méthyl]-carbamoyl)-desferrioxamine B,
la N-(éthoxycarbonyl-méthoxycarbonyl)-0,0′,0″-tri-(N-[2-éthoxycarbonyl-éthyl]-carbamoyl)-desferrioxamine B,
la N-(éthoxycarbonyl-méthoxycarbonyl)-O,O′,O″-tri-(N-[éthoxycarbonyl-méthyl]-carbamoyl)-desferrioxamine B et
la N-(tert.butyloxycarbonyl-O,O′,O″-tri-(N-[2-éthoxycarbonyl-éthyl]-carbamoyl)-desferrioxamine B.

10. Composé selon l'une des revendications 1 à 9, destiné à être utilisé dans un procédé pour le traitement thérapeutique du corps humain ou animal.

11. Composition pharmaceutique contenant, comme substance active, à côté du support pharmaceutique, un composé de formule I selon l'une des revendications 1 à 9 ou un sel pharmaceutiquement utilisable d'un tel composé ayant des propriétés salifiables.

12. Utilisation d'un composé selon l'une des revendications 1 à 9 pour la préparation de compositions pharmaceutiques destinées à être utilisées pour le traitement des états pathologiques chez les êtres à sang chaud incluant l'homme, liés à un excès en fer (III) ou en aluminium dans le corps ou causés par des organismes pathogènes dépendant du fer (III).

13. Procédé pour la préparation d'un composé de formule I selon la revendication 1 ou d'un sel d'un tel composé ayant des propriétés salifiables, caractérisé

a) en ce que l'on fait réagir un dérivé de la desferrioxamine B de formule IV

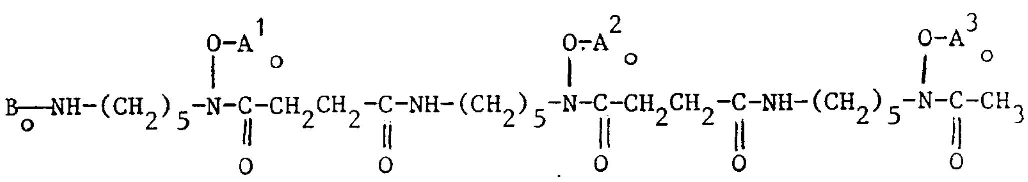

(IV)

où B° représente un reste acyle Ac, différent de alkyl-O-($CH_2$-$CH_2$-O-)$_q$CO-, q représentant un nombre plus grand que 5 ou un groupe protecteur de l'amino et où au moins l'un des symboles $A^1$, $A^2$ et $A^3$ représente l'hydrogène et les symboles $A_o^1$, $A_o^2$ et $A_o^3$ restants représentent un reste acyle Ac défini ci-dessus, avec un ester d'acide isocyanatocarboxylique de formule III

$$O=C=N\text{-}Alk\text{-}CO\text{-}O\text{-}R_a^1 \qquad (III)$$

où $R_a^1$ et Alk ont les significations données ci-dessus, et dans le cas où dans le reste Alk sont présents des groupes amino ou hydroxy, ceux-ci sont protégés par des groupes protecteurs facilement clivables et en ce que l'on clive les groupes protecteurs présents ou

b) en ce que pour la préparation d'un composé de formule I où B représente un reste acyle, différent de alkyl-O-($CH_2$-$CH_2$-O-)$_q$CO-, q représentant un nombre plus grand que 5, on acyle un composé de formule I où B représente l'hydrogène ou un groupe silyle, et, si désiré, en ce que l'on transforme, après réalisation des procédés a) ou b), le composé libre de formule I obtenu présentant des propriétés salifiables en l'un de ses sels ou en ce qu'on libère le composé de formule I de l'un de ses sels.

**Revendications pour les Etats contractants suivants : ES, GR**

1. Procédé pour la préparation d'un composé de formule I

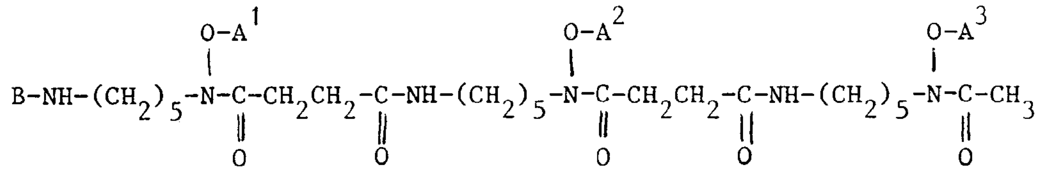

(I)

dans laquelle au moins l'un des restes $A^1$, $A^2$ et $A^3$ représente un reste carbamoyl de formule partielle -CO-NH-Alk-CO-O-$R^1_a$ (II) où $R^1_a$ représente un alkyle en $C_1$-$C_4$ ou un alcényle en $C_2$-$C_4$ et Alk représente un alkylène en $C_1$-$C_7$ non substitué ou substitué par l'hydroxyle, un alcanoyloxy en $C_1$-$C_4$, un amino, un alcoxy en $C_1$-$C_4$ carbonyle, carbamoyle, un phényle, un hydroxyphényle, le méthoxyphényle ou l'indolyle, et les symboles $A^1$, $A^2$ et $A^3$ restants représentent indépendamment l'un de l'autre l'hydrogène ou un reste acyle Ac dérivé d'un acide carboxylique et B représente l'hydrogène ou un reste acyle Ac différent d'un alkyl-O-($CH_2$-$CH_2$-O-)$_q$CO- où q représente un nombre plus grand que 5 ou d'un sel d'un tel composé ayant des propriétés salifiables, caractérisé

a) en ce que l'on fait réagir un dérivé de la desferrioxamine B IV

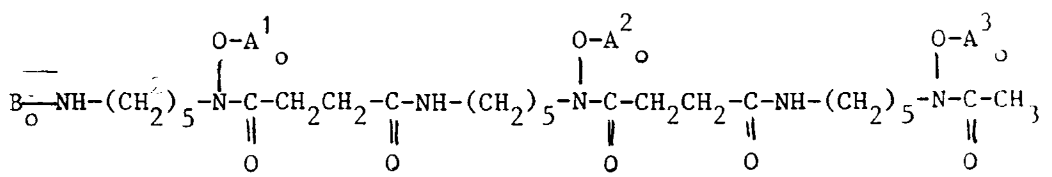

(IV)

où B° représente un reste acyle Ac, différent de alkyl-O-($CH_2$-$CH_2$-O-)$_q$CO-, q représentant un nombre plus grand que 5 ou un groupe protecteur de l'amino et où au moins l'un des symboles $A^1_o$, $A^2_o$ et $A^3_o$ représente l'hydrogène et les symboles $A^1_o$, $A^2_o$ et $A^3_o$ restants représentent un reste acyle Ac défini ci-dessus, avec un ester d'acide isocyanatocarboxylique de formule III

O=C=N-Alk-CO-O-$R^1_a$ (III)

où $R^1_a$ et Alk ont les significations données ci-dessus, et dans le cas où dans le reste Alk sont présents des groupes amino ou hydroxy, ceux-ci sont protégés par des groupes protecteurs facilement clivables et en ce que l'on clive les groupes protecteurs présents ou

b) en ce que pour la préparation d'un composé de formule I où B représente un reste acyle, différent de alkyl-O-($CH_2$-$CH_2$-O-)$_q$CO-, q représentant un nombre plus grand que 5, on acyle un composé de formule I où B représente l'hydrogène ou un groupe silyle et $A^1$, $A^2$ et $A^3$ ont les significations précitées, et si désiré, en ce que l'on transforme après réalisation des procédés a) ou b), le composé libre de formule I obtenu présentant des propriétés salifiables en l'un de ses sels ou en ce qu'on libère le composé de formule I de l'un de ses sels.

2. Procédé selon la revendication 1, caractérisé en ce que l'on choisit les produits de départ de telle façon que l'on prépare un composé de formule I où $A^1$, $A^2$ et $A^3$ représentent chacun un seul et même reste carbamoyle de formule partielle II et B représente un reste acyle, différent alkyl -O-($CH_2$-$CH_2$-O-)$_q$CO-, q représentant un nombre plus grand que 5.

3. Procédé selon la revendication 1 ou 2, caractérisé en ce que l'on choisit les produits de départ de telle façon que l'on prépare un composé de formule I dans laquelle B représente un alcanoyle en $C_2$-$C_{10}$, un (alcoxy en $C_1$-$C_9$)-carbonyle, un dialkyle inférieur amino-carboynyle, un mono-, di- ou tri-oxa-alcoxycarbonyle linéaire ayant jusqu'à 18 chaînons ou un ω-(alcoxy en $C_1$-$C_4$ carbonyl)-alcoxy inférieur carbonyle.

4. Procédé selon l'une des revendications 1 à 3, caractérisé en ce que l'on choisit les produits de départ de telle façon que l'on prépare un composé de formule I où dans le reste de formule partielle II $R^1_a$ représente un reste alkyle en $C_1$-$C_4$ linéaire et Alk représente un reste alkylène en $C_1$-$C_7$ linéaire dont les valences libres partent des deux atomes de C terminaux.

5. Procédé selon la revendication 1, caractérisé en ce que l'on choisit les produits de départ de telle façon que l'on prépare un composé de formule I dans laquelle $A^1$, $A^2$ et $A^3$ ont la même signification et représentent un reste carbamoyle de formule partielle II où $R_a^1$ représente un alkyle en $C_1$-$C_4$ et Alk un alkylène en $C_1$-$C_5$ et B un 2-(alcoxy en $C_2$-$C_4$)-éthoxycarbonyle, un 2-[2-(alcoxy en $C_2$-$C_4$)-éthoxy]-éthoxycarbonyle ou l'éthoxycarbonylméthoxycarbonyle.

6. Procédé selon la revendication 1, caractérisé en ce que l'on choisit les produits de départ de telle façon que l'on prépare un composé de formule I dans laquelle $A^1$, $A^2$ et $A^3$ ont la même signification et représentent un reste carbamoyle de formule partielle II où $R_a^1$ représente un alkyle en $C_1$-$C_4$ et Alk le méthylène ou l'éthylène et B un 2-(alcoxy en $C_2$-$C_4$)-éthoxycarbonyle, un 2-[2-(alcoxy en $C_2$-$C_4$)-éthoxy]-éthoxycarbonyle, l'éthoxycarbonylméthoxycarbonyle ou un (alcoxy en $C_1$-$C_4$)-carbonyle.

7. Procédé selon l'une des revendications 1 à 3 et 5, caractérisé en ce que l'on choisit les produits de départ de telle façon que l'on prépare un composé de formule I où dans le reste de formule partielle II $R_a^1$ représente un alkyle en $C_1$-$C_4$ linéaire et Alk un 1,1-alkylène en $C_1$-$C_5$ linéaire ou ramifié une fois.

8. Procédé selon l'une des revendications 1 à 5, caractérisé en ce que l'on choisit les produits de départ de telle façon que l'on prépare un composé de formule I où dans le reste de formule partielle II $R_a^1$ représente un alkyle en $C_1$-$C_4$ linéaire et Alk le méthylène ou l'éthylène.

9. Procédé selon la revendication 1, caractérisé en ce que l'on choisit les produits de départ de telle façon que l'on prépare la N-(2-butoxy-éthoxy-carbonyl)-O,O',O"-tri-(N-[éthoxycarbonylméthyl]-carbamoyl)-desferrioxamine B.

10. Procédé selon la revendication 1, caractérisé en ce que l'on choisit les produits de départ de telle façon que l'on prépare la N-(2-butoxy-éthoxy-carbonyl)-0,0',0"-tri-(N-[2-éthoxycarbonyl-éthyl]-carbamoyl)-desferrioxamine B.

11. Procédé selon la revendication 1, caractérisé en ce que l'on choisit les produits de départ de telle façon que l'on prépare la N-[2-(2-éthoxy-éthoxy)-éthoxycarbonyl]-0,0',0"-tri-(N-[éthoxycarbonylméthyl]-carbamoyl)-desferrioxamine B.

12. Procédé selon la revendication 1, caractérisé en ce que l'on choisit les produits de départ de telle façon que l'on prépare la N-[2-(2-éthoxy-éthoxy)-éthoxycarbonyl]-0,0',0"-tri-(N-[2-éthoxycarbonyl-éthyl]-carbamoyl)-desferrioxamine B.

13. Procédé selon la revendication 1, caractérisé en ce que l'on choisit les produits de départ de telle façon que l'on prépare un composé choisi dans le groupe constitué par
la N-[2-(2-butoxy-éthoxy)-éthoxy-carbonyl]-O,O',O"-tri(N-[2-éthoxycarbonyl-éthyl]-carbamoyl)-desferrioxamine B,
la N-[2-(2-butoxy-éthoxy)-éthoxy-carbonyl]-O,O',O"-tri(N-[éthoxycarbonyl-méthyl]-carbamoyl)-desferrioxamine B,
la N-(éthoxycarbonyl-méthoxycarbonyl)-O,O',O"-tri-(N-[2-éthoxycarbonyl-éthyl]-carbamoyl)-desferrioxamine B,
la N-(éthoxycarbonyl-méthoxycarbonyl)-O,O',O"-tri-(N-[éthoxycarbonyl-méthyl]-carbamoyl)-desferrioxamine B et
la N-(tert.butyloxycarbonyl-O,O'-O"-tri-(N-[2-éthoxycarbonyl-éthyl]-carbamoyl)-desferrioxamine B.